# EUROPEAN PATENT SPECIFICATION

(11) **EP 4 244 385 B1**
(45) Date of publication and mention of the grant of the patent: **01.01.2025**
(21) Application number: 21819349.8
(22) Date of filing: 10.11.2021
(51) Int. Cl.: C12Q 1/6853, C12Q 1/6844

(54) **LOOP MEDIATED ISOTHERMAL NUCLEIC ACID AMPLIFICATION (LAMP) USING LNA-MODIFIED PRIMERS AND A METAL-BASED COLORIMETRIC METHOD FOR DETECTING AN AMPLIFICATION PRODUCT**
LOOP-VERMITTELTER ISOTHERMER AMPLIFIKATION (LAMP) UNTER VERWENDUNG VON LNA-MODIFIZIERTEN PRIMERN, UND EIN METALLBASIERTES COLORIMETRISCHES VERFAHREN ZUR DETEKTION VON AMPLIFIKATIONSPRODUKTE
AMPLIFICATION ISOTHERME À MÉDIATION EN BOUCLE (LAMP) À LAIDE DES AMORCES MODIFIES PAR LNA, ET PROCÉDÉ DE COLORIMÉTRIE À BASE DE MÉTAL POUR LA DÉTECTION D'UN PRODUIT D'AMPLIFICATION

(30) Priority: 10.11.2020 EP 20206643; 02.12.2020 US 202063120652 P
(43) Date of publication of application: 20.09.2023
(73) Proprietor: MultiplexDX, s.r.o., 84104 Bratislava (SK)
(72) Inventor: CEKAN, Pavol, 84104 Bratislava (SK); PAUL, Evan, 84104 Bratislava (SK); SZOBI, Adrián, 84104 Bratislava (SK); VOJTASSÁKOVÁ, Nina, 84104 Bratislava (SK); BURANOVSKÁ, Katarina, 84104 Bratislava (SK)
(74) Representative: Schiweck Weinzierl Koch Patentanwälte Partnerschaft mbB
(86) International application number: PCT/EP2021/081230
(87) International publication number: WO 2022/101259

(56) References cited:
- WO-A1-2017/079696
- WO-A1-2019/234252
- CN-A- 107 338 289
- CN-A- 107 841 536
- JP-A- 2004 283 161
- US-B1- 10 968 493
- P BREDBACKA: "PROGRESS ON METHODS OF GENE DETECTION IN PREIMPLANTATION EMBRYOS", THERIOGENOLOGY, vol. 55, 1 January 2001 (2001-01-01), pages 23 - 34, XP055406916, DOI: 10.1016/S0093-691X(00)00443-X
- MAERTENS O ET AL: "Real-time quantitative allele discrimination assay using 3' locked nucleic acid primers for detection of low-percentage mosaic mutations", ANALYTICAL BIOCHEMISTRY, ACADEMIC PRESS, AMSTERDAM, NL, vol. 359, no. 1, 1 December 2006 (2006-12-01), pages 144 - 146, XP024942281, ISSN: 0003-2697, [retrieved on 20061201], DOI: 10.1016/J.AB.2006.07.039
- ITONAGA MASAHIRO ET AL: "Novel Methodology for Rapid Detection of KRAS Mutation Using PNA-LNA Mediated Loop-Mediated Isothermal Amplification", PLOS ONE, vol. 11, no. 3, 21 March 2016 (2016-03-21), pages e0151654, XP055797130, DOI: 10.1371/journal.pone.0151654
- NORIHIRO TOMITA ET AL: "Loop-mediated isothermal amplification (LAMP) of gene sequences and simple visual detection of products", NATURE PROTOCOLS, vol. 3, no. 5, 1 April 2008 (2008-04-01), GB, pages 877 - 882, XP055221080, ISSN: 1754-2189, DOI: 10.1038/nprot.2008.57
- ZHANG MENGYAO ET AL: "Visual detection for nucleic acid-based techniques as potential on-site detection methods. A review", ANALYTICA CHIMICA ACTA, ELSEVIER, AMSTERDAM, NL, vol. 1099, 26 November 2019 (2019-11-26), pages 1 - 15, XP086004541, ISSN: 0003-2670, [retrieved on 20191126], DOI: 10.1016/J.ACA.2019.11.056
- WANG DEGUO ET AL: "Development of a real-time loop-mediated isothermal amplification (LAMP) assay and visual LAMP assay for detection of African swine fever virus (ASFV)", JOURNAL OF VIROLOGICAL METHODS, ELSEVIER BV, NL, vol. 276, 11 November 2019 (2019-11-11), XP086038872, ISSN: 0166-0934, [retrieved on 20191111], DOI: 10.1016/J.JVIROMET.2019.113775

## Description

This application contains a Sequence Listing in computer readable form.

### TECHNICAL FIELD OF THE INVENTION

The present invention relates to a loop mediated isothermal amplification (LAMP) method characterized in that the F3 nucleotide sequence and/or said B3 nucleotide sequence comprises one or more locked nucleic acid (LNA) nucleotides which are located within the first third of said F3 nucleotide sequence or said B3 nucleotide sequence, respectively. The present invention further relates to an *in vitro* method for detecting a nucleic acid sequence amplification product characterized by the use of a metallochromic indicator, preferably 5-Br-PAPS, and metal ions, preferably Zn²⁺ ions. The present invention further relates to a kit for carrying out the methods of the invention and a use of the kit.

### BACKGROUND

Since the revolutionary development of the polymerase chain reaction (PCR) in the 1980s, nucleic acid amplification tests (NAATs) have become an indispensable tool throughout the entire life sciences field, and have even grown to be the gold standard of nucleic acid analysis, especially in clinical applications, but also for food quality control and environmental monitoring. A remarkable trend, emerging between 1995 and 2005, can also be observed in the development of isothermal NAATs, which was provoked by the limitations of PCR. The complex and expensive devices required for thermal cycling and real-time detection during PCR restrict the use of this amplification method. Isothermal NAATs enable amplification reactions at constant and moderate temperatures. Simple and low-cost devices, as well as fast processing times compared to PCR, make isothermal NAATs increasingly attractive and open up new application opportunities in the field of point-of-care (POC)/point-of-need (PON) testing.

Loop-mediated isothermal amplification (LAMP), which was first described by Notomi et al. (2000), Nucleic Acids Res., 28(12):e63, see also EP 1020534 B2, is one of the methods for performing NAATs. Also WO2019234353 disclosed amplification by LAMP. CN107338289 and CN107841536 used locked nucleic acids in LAMP inner primers.

However, there is an ongoing need to improve LAMP, especially by increasing the specificity of the primers. Further, there is an ongoing need for improved detection methods that can be used in NAATs. The technical problem therefore is to comply with this need.

### SUMMARY OF THE INVENTION

The technical problem is solved by the subject-matter as defined in the claims.

Accordingly, the present invention relates to a loop mediated isothermal amplification (LAMP) method, comprising synthesizing a nucleic acid sequence from a template nucleic acid sequence in a reaction mixture comprising
(i) a first inner primer (FIP) nucleotide sequence and a second inner primer (BIP) nucleotide sequence,
(ii) a first outer primer (F3) nucleotide sequence and a second outer primer (B3) nucleotide sequence, wherein said F3 nucleotide sequence and/or said B3 nucleotide sequence comprises one or more locked nucleic acid (LNA) nucleotides which are located within the first third of said F3 nucleotide sequence or said B3 nucleotide sequence, respectively, wherein said first third is the first third of a nucleotide sequence in 5'- to 3'-orientation,
(iii) a DNA polymerase catalyzing a strand-displacement-type reaction of synthesizing a complementary nucleic acid strand from said template nucleic acid, and
(iv) nucleotides serving as substrates for said DNA polymerase at such a temperature that at least FIP nucleotide sequence and BIP nucleotide sequence can form stable base pairing with its complementary nucleotide sequences comprised by said template nucleic acid while the DNA polymerase activity can be maintained, thereby synthesizing a nucleic acid sequence.

The present invention further relates to an *in vitro* method for detecting a nucleic acid sequence amplification product, comprising
(a) contacting a reaction mixture comprising a template nucleic acid sequence, primer nucleotide sequences, nucleotides and a polymerase capable of amplifying said nucleic acid molecule with a metallochromic indicator and a transition or post-transition metal ion, said metallochromic indicator and said transition or post-transition metal ion building a complex, but not building a complex with magnesium;
(b) amplifying said nucleic acid sequence under suitable conditions to obtain a nucleic acid sequence amplification product;
(c) detecting a change in spectral or fluorescent properties of the complex resulting from amplification of the target nucleic acid, wherein formation of said complex comprising said metallochromic indicator and said transition or post-transition metal ion leads to a color change of the solution; wherein the amplification is performed by the LNA-LAMP method of the present invention.

The present invention further relates to a kit for the synthesis of a nucleic acid sequence by loop mediated isothermal amplification (LAMP) on a template nucleic acid sequence, comprising
(i) a first outer primer (F3) nucleotide sequence and a second outer primer (B3) nucleotide sequence, each of which comprises locked nucleic acid (LNA) nucleotides, which are located within the first third of said F3 nucleotide sequence or said B3 nucleotide sequence, respectively, wherein said first third is the first third of a nucleotide sequence in 5'- to 3'-orientation,
(ii) a first inner primer (FIP) nucleotide sequence and a second inner primer (BIP) nucleotide sequence,
(iii) a DNA polymerase catalyzing a strand-displacement-type reaction of synthesizing a complementary nucleic acid strand from said template nucleic acid,
(iv) nucleotides serving as substrates for said DNA polymerase, and optionally
(v) a metallochromic indicator, preferably 5-Br-PAPS; and/or optionally
(vi) metal ions, preferably Zn²⁺ ions.

The present invention further relates to the use of the kit of the invention for performing the method of the LAMP method of the invention.

### BRIEF DESCRIPTION OF THE DRAWINGS

The invention will be better understood with reference to the detailed description when considered in conjunction with the non-limiting examples and the accompanying drawings, respectively. The Figures show:

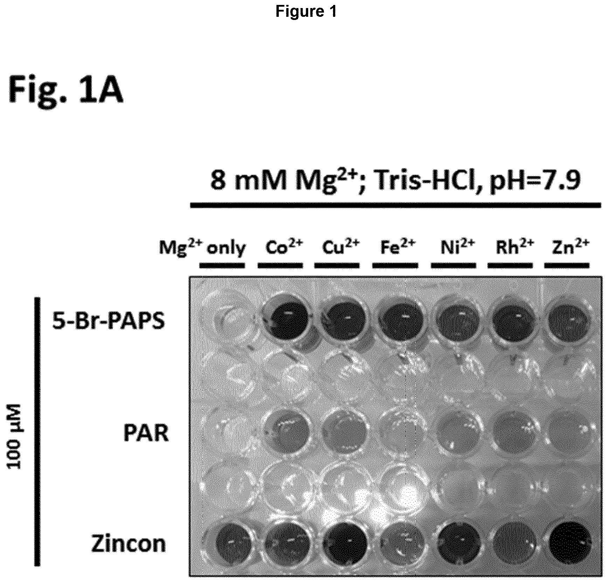

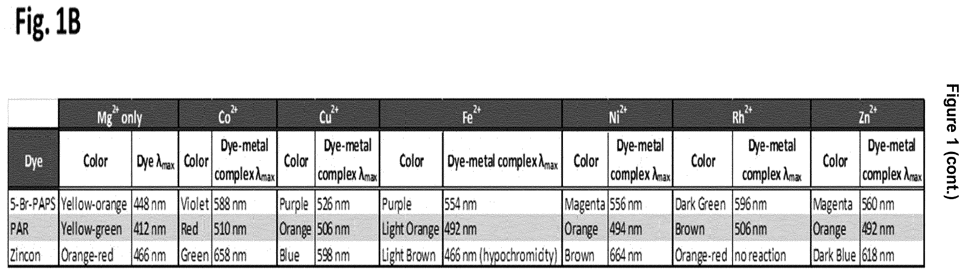

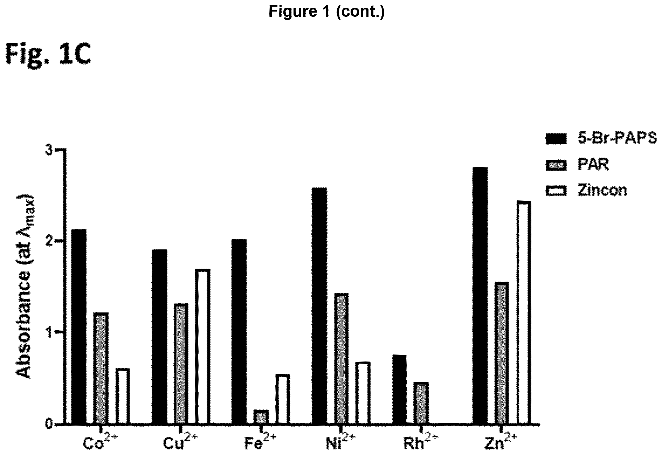

**Figure 1** shows the feasibility testing of preferred chelatometric dyes (5-Br-PAPS, PAR, zincon) and their formation of complexes with preferred metal ions (Co²⁺, Cu²⁺, Fe²⁺, Ni²⁺, Rh²⁺, Zn²⁺) in the presence of Mg²⁺. Profound color change (Fig. 1A-B) indicating complex formation was observed for almost all combinations of dyes and metal ions. Spectrophotometric analysis further demonstrated (Fig. 1C) that complexes of the preferred dye 5-Br-PAPS show the highest average absorbance values for the same concentration of the dye.

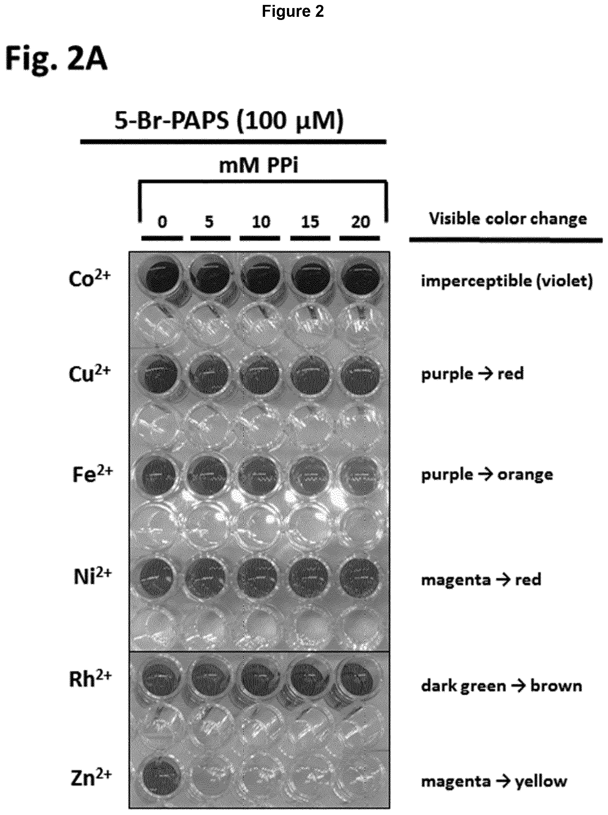

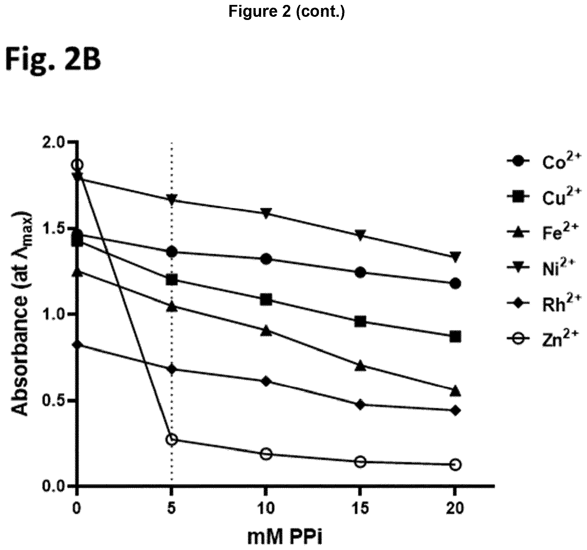

**Figure 2** shows the stability of the 5-Br-PAPS complexes with preferred metal ions with respect to pyrophosphate (PPi) concentrations. Both visual (Fig 2A) and spectral (2B) changes showed that progressively increasing concentrations of PPi (0-20 mM, left-to-right) were able to destabilize the complex leading to an observable color change. The complex of 5-Br-PAPS and Zn²⁺ was by far the most sensitive to PPi highlighting its potential in PPi detection during ongoing nucleic acid amplification reactions.

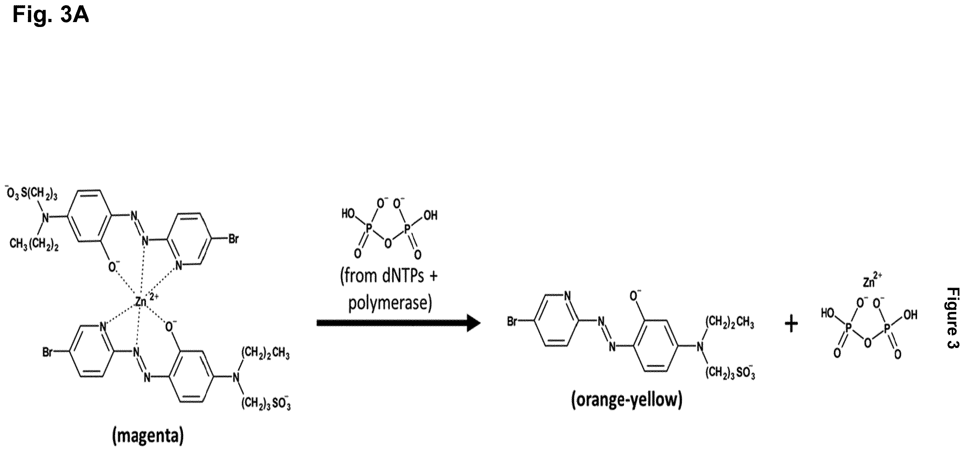

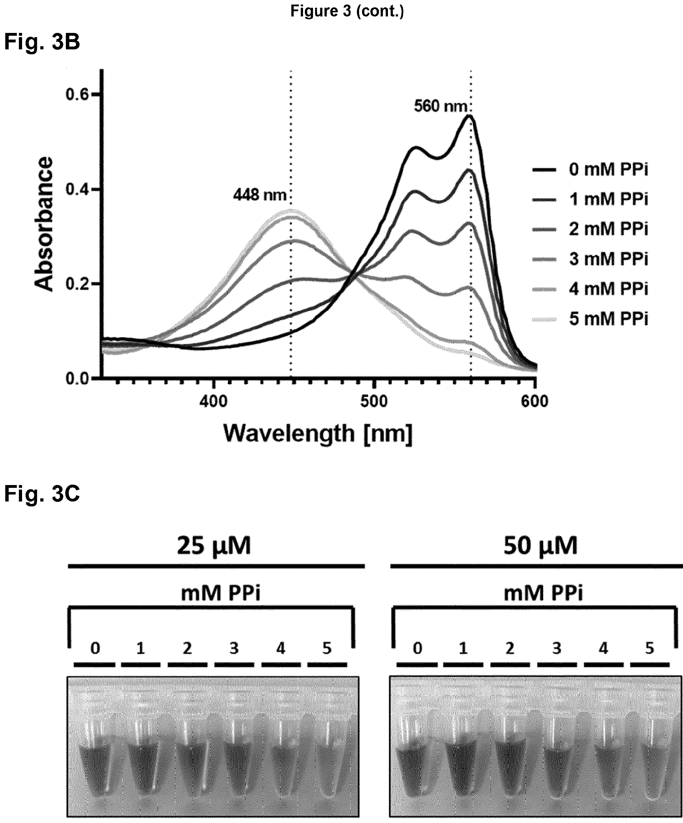

**Figure 3** shows the proposed mechanism (Fig. 3A) as well as supporting data behind the color change of the Zn²⁺/5-Br-PAPS detection reagent during amplification reactions. Specifically, both spectrophotometric (Fig. 3B) and visual (Fig. 3C) data show that either 25 or 50 µM of the reagent changes color from magenta to orange-yellow (from left to right/from darker to brighter) in a pyrophosphate (PPi) concentration-dependent manner, which is normally produced by the action of a DNA polymerase that incorporates a nucleotide into a nucleic acid strand.

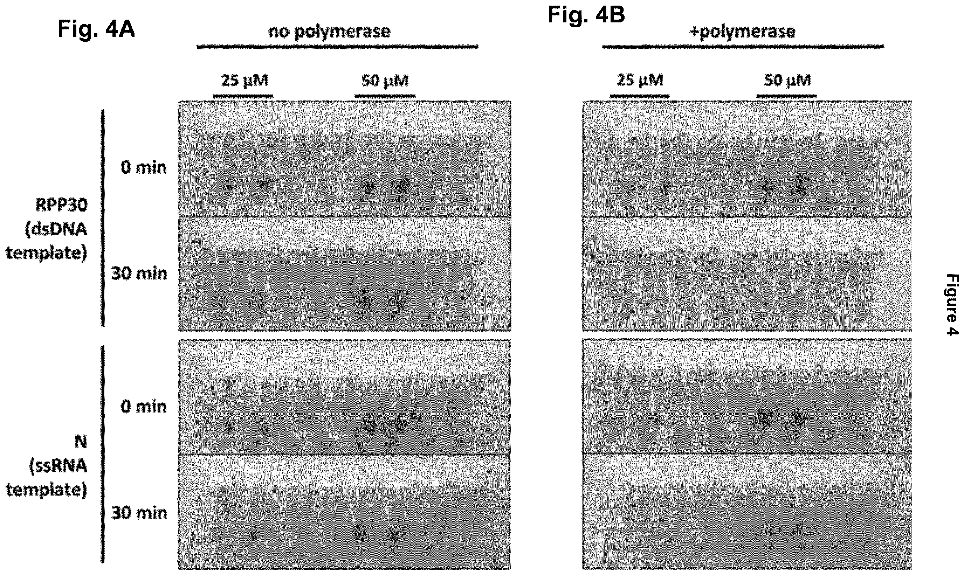

**Figure 4** shows the color transition of the Zn²⁺/5-Br-PAPS detection reagent before and after a LAMP amplification reaction. LAMP and RT-LAMP was used to amplify human RPP30 double-stranded (ds) DNA (Fig. 4A) and SARS-CoV-2 N gene single-stranded (ss) RNA (Fig. 4B), respectively, with either 25 or 50 µM of the Zn²⁺/5-Br-PAPS detection reagent in the reaction mix. After 30 minutes, a clear color change in samples with Bst 2.0 polymerase can be observed irrespective of the template type employed demonstrating the color change is specific to amplification of a nucleic acid template and not due to heating.

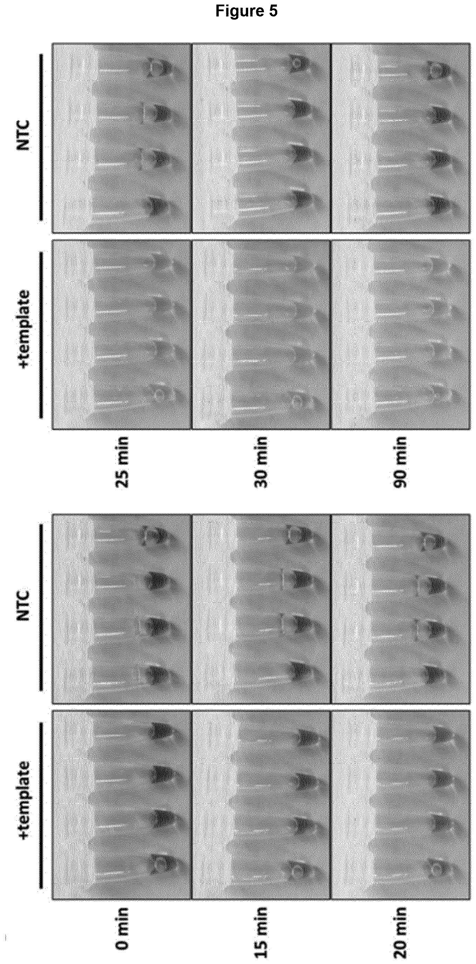

**Figure 5** shows a time course of the Zn²⁺/5-Br-PAPS detection reagent color transition. Here, LAMP was used to amplify human RPP30 dsDNA over the course of 90 minutes. A progressive change of color from magenta through red to orange-yellow was observed but only in samples with template dsDNA. Samples positive for amplification were easily distinguishable from NTC samples even before the LAMP reactions reached completion. Even though the color transition was complete by the 25-minute mark, the detection reagent retained its color both in NTC and template-containing samples throughout the whole 90-minute period.

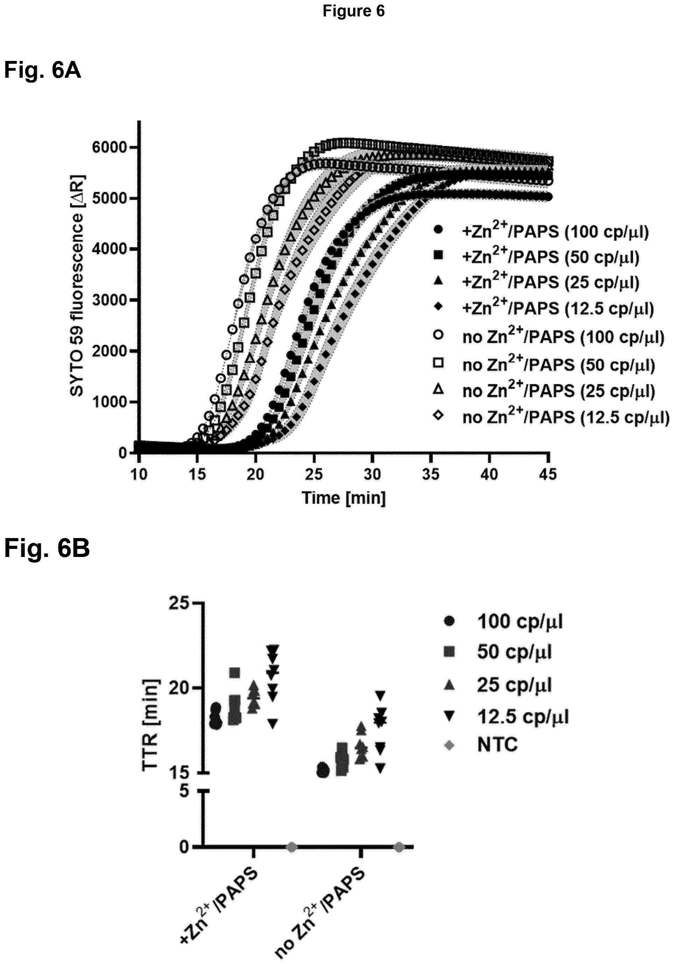

**Figure 6** shows the effects of the Zn²⁺/5-Br-PAPS detection reagent on key LAMP amplification parameters. Here, 45-minute RT-LAMP reactions were performed targeting SARS-CoV-2 RdRP gene with or without the Zn²⁺/5-Br-PAPS detection reagent. A range of starting template amount was used, and amplification was monitored fluorescently with the nucleic acid-intercalating dye, SYTO 59. Inclusion of 50 µM of the Zn²⁺/5-Br-PAPS detection reagent did not significantly change the amplification curves (Fig. 6A) while time-to-reaction (TTR) was prolonged only marginally (Fig. 6B).

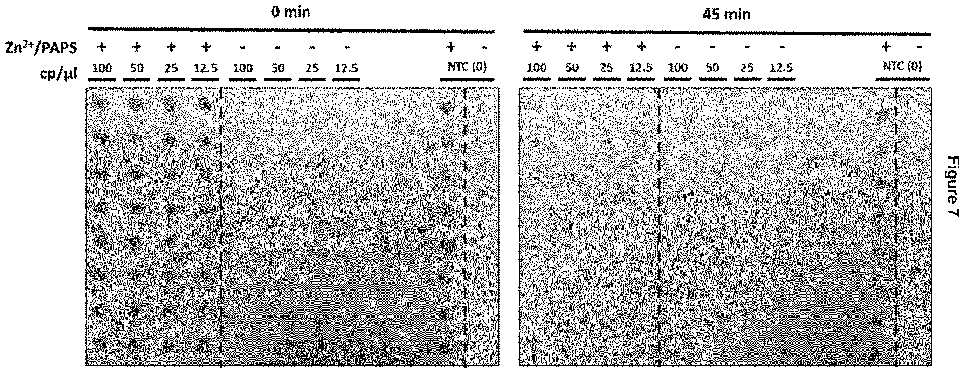

**Figure 7** is an annotated image of the same experiment as described in Figures 6A-B. After LAMP amplification, in reactions with the Zn²⁺/5-Br-PAPS detection reagent, the color of NTC reactions stayed magenta while all samples changed color to orange-yellow irrespective of the starting template amount.

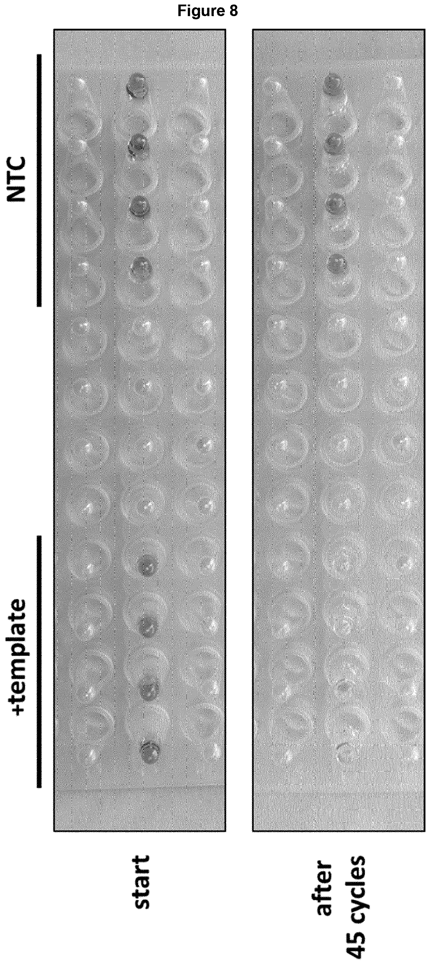

**Figure 8** shows the performance of the Zn²⁺/5-Br-PAPS detection reagent using the PCR amplification method. After 45 cycles, reactions containing 25 µM of the Zn²⁺/5-Br-PAPS detection reagent changed color from magenta to orange-yellow but only in samples containing dsDNA template where subsequent amplification of human RPP30 occured.

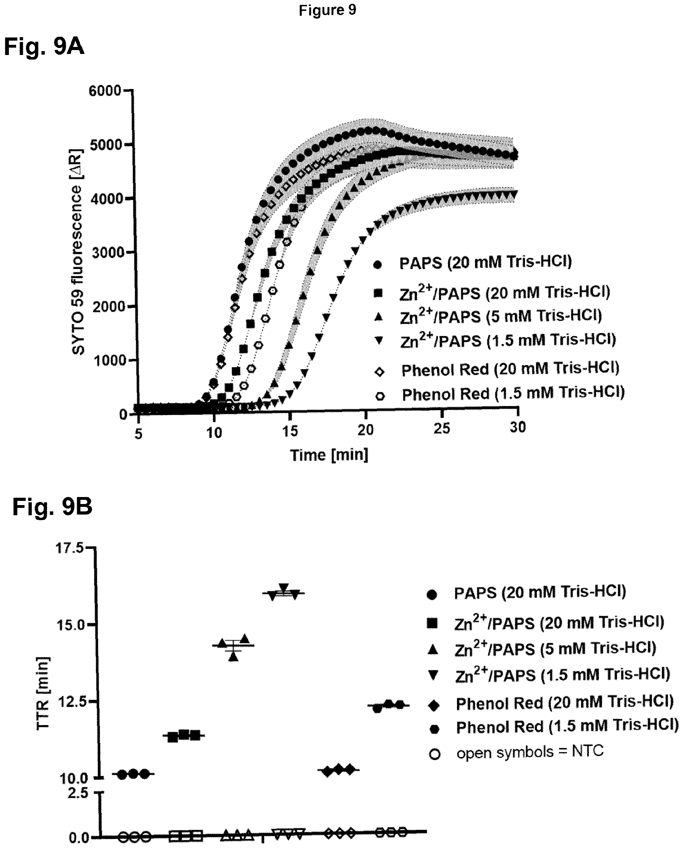

**Figure 9** shows LAMP amplification performance of master mixes with either Zn²⁺/5-Br-PAPS, phenol red (pH-sensitive dye) or 5-Br-PAPS only. A 30-minute LAMP reaction was used to amplify human RPP30 while amplification was monitored real-time with the nucleic acid-intercalating dye, SYTO 59. All variants showed amplification (Fig. 9A) irrespective of Tris-HCl concentration but higher Tris-HCl concentrations showed lower time-to-reaction (TTR, Fig. 9B) for both Zn²⁺/5-Br-PAPS and phenol red.

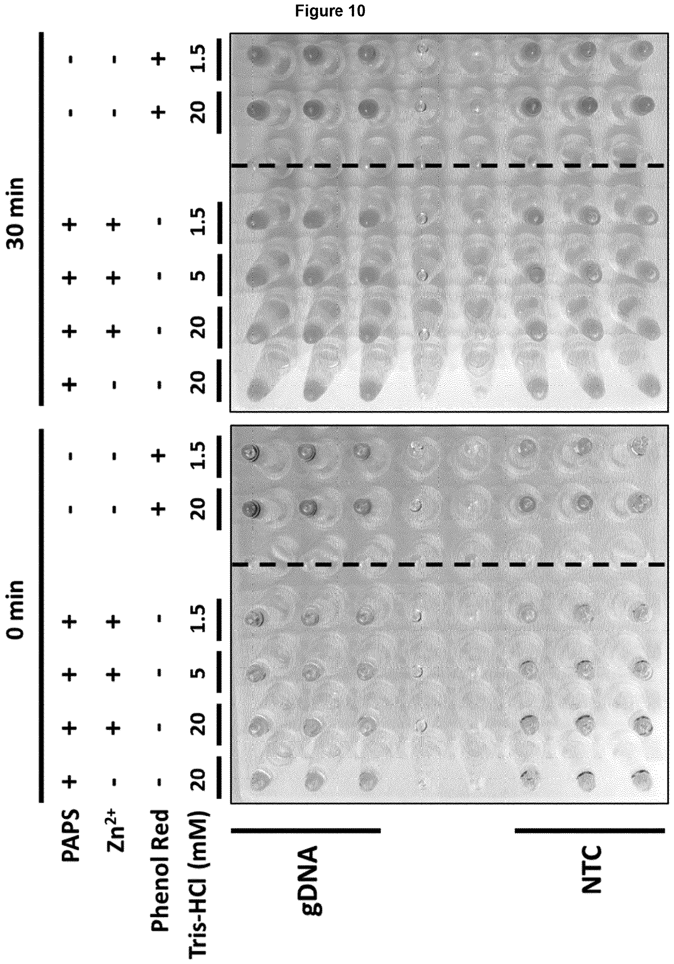

**Figure 10** is an annotated image of the same experiment as described in Figures. 9A-B. Unlike pH-dependent detection exemplified by using phenol red as the colorimetric detector (metallochromic indicator), the Zn²⁺/5-Br-PAPS detection reagent still changed color to orange-yellow upon successful LAMP amplification even in the presence of up to 20 mM Tris-HCl pH=7.9. This color change was dependent on the presence of Zn²⁺ as 5-Br-PAPS by itself failed to change color.

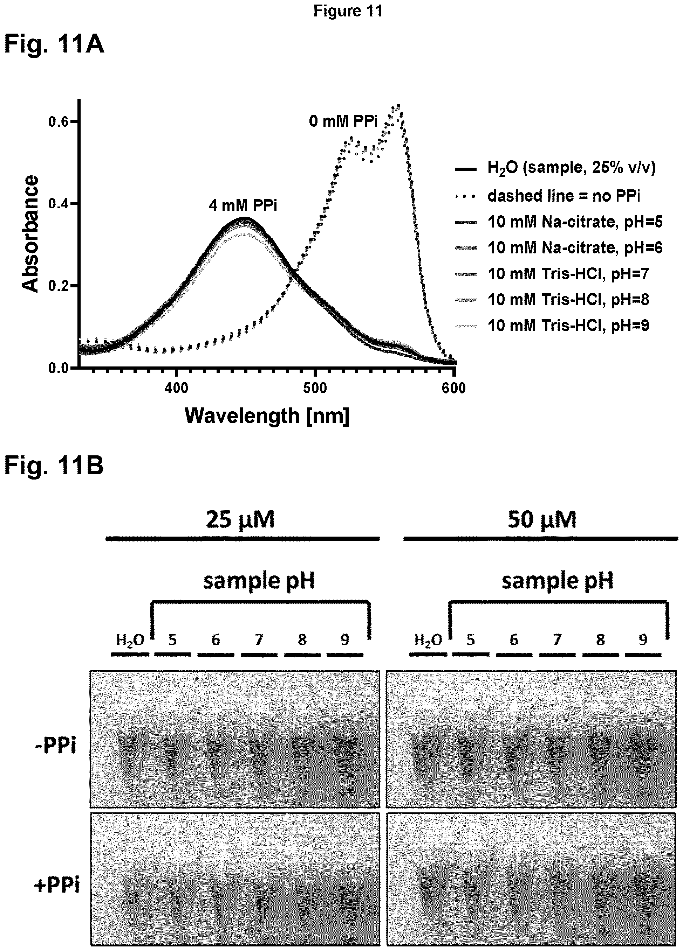

**Figure 11** shows that the sodium pyrophosphate-induced color transition of the Zn²⁺/5-Br-PAPS detection reagent is retained even with the addition of different pH buffers. Here, mock LAMP samples were prepared in such a way that samples with no pyrophosphate (PPi) represent negative/pre-amplification samples while those with pyrophosphate mimic samples where amplification did take place. Both spectrophotometric (Fig. 11A) and visual (Fig. 11B) data show that the behavior of the Zn²⁺/5-Br-PAPS detection reagent is unchanged by the addition of 25 % (v/v) of 10 mM buffers with various pH values.

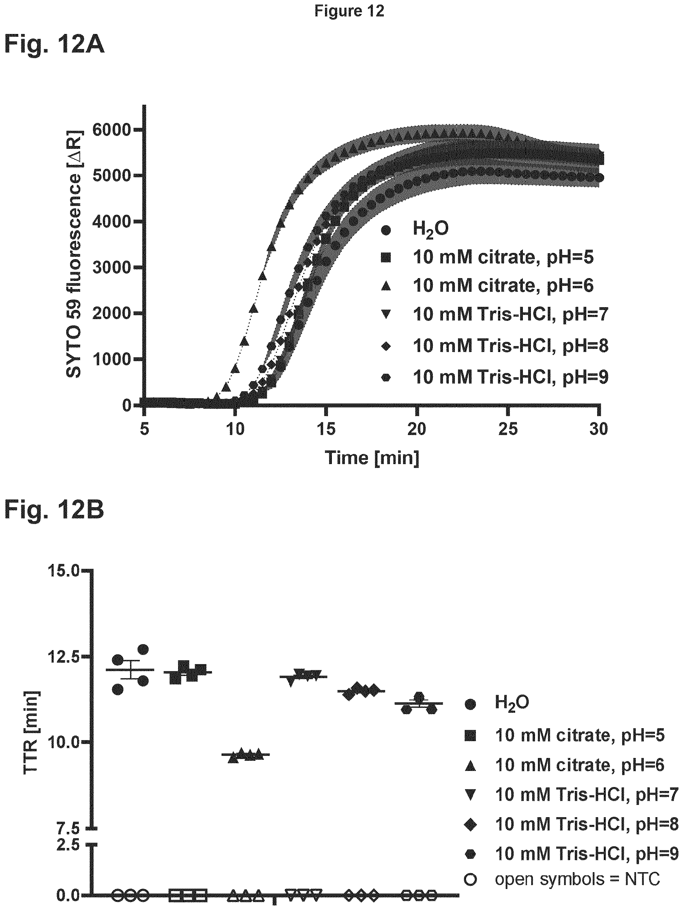

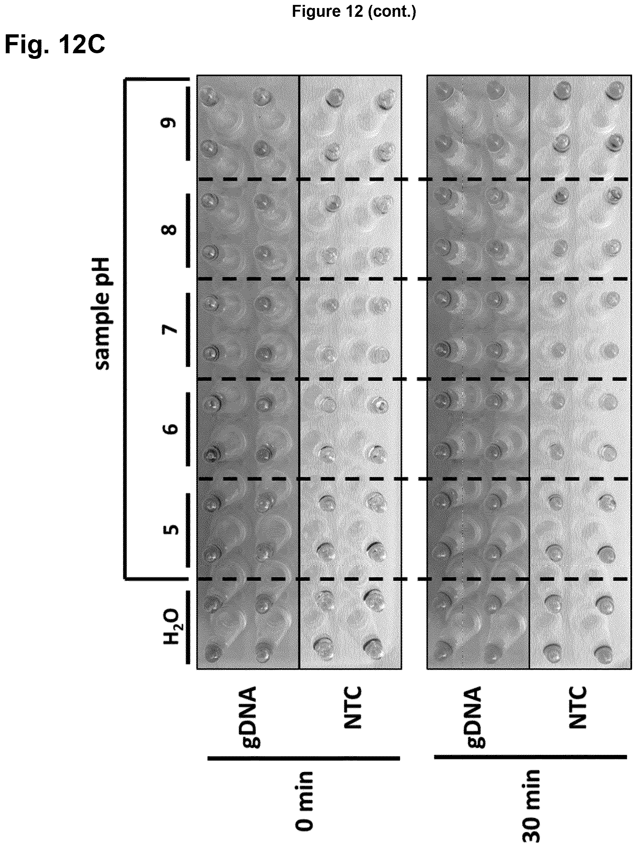

**Figure 12** shows the performance of the Zn²⁺/5-Br-PAPS detection reagent in LAMP amplification reactions with input samples buffered to different pH levels. A 30-minute LAMP amplification reaction was performed with a primer set targeting human RPP30 genomic DNA (gDNA) while amplification was monitored real-time with the nucleic acid-intercalating dye, SYTO 59. Samples with template prepared in different pH buffers did not negatively impact amplification (Fig. 12A), time-to-reaction (TTR, Fig. 12B) or the resulting color change (Fig. 12C).

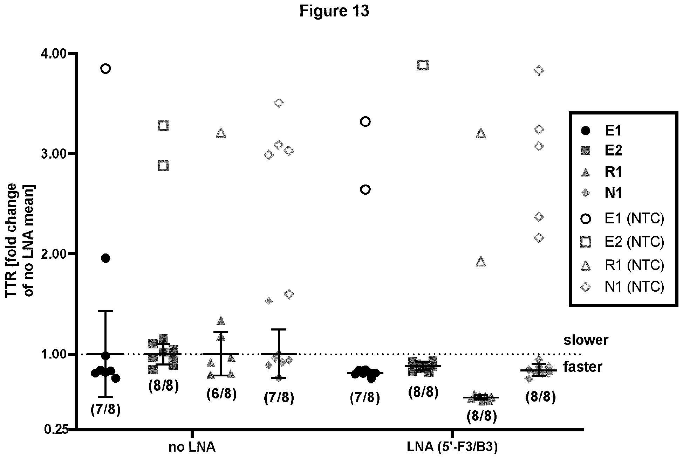

**Figure 13** shows that the LNA modification of the outer F3/B3 primers in LAMP leads to a universal improvement in LAMP reaction performance. Four different F3/B3 primer sets targeting 3 distinct SARS-CoV-2 genes demonstrate faster reaction kinetics (shorter time-to-reaction, TTR), smaller amplification variance (standard deviation, SD; data point spread) and increased sensitivity (more replicates amplified). The numbers in parenthesis denote the number of replicates amplified out of the total number of replicates. The dotted line represents the mean TTR of the no LNA primer sets; data points above and below the dotted line are slower and faster, respectively, than the mean TTR of the no LNA primer set. E1 and E2, SARS-CoV-2 Envelope gene primer sets 1 and 2; R1, SARS-CoV-2 RNA-dependent RNA Polymerase gene primer set 1; N1, SARS-CoV-2 Nucleoprotein gene primer set 1; NTC, no template control.

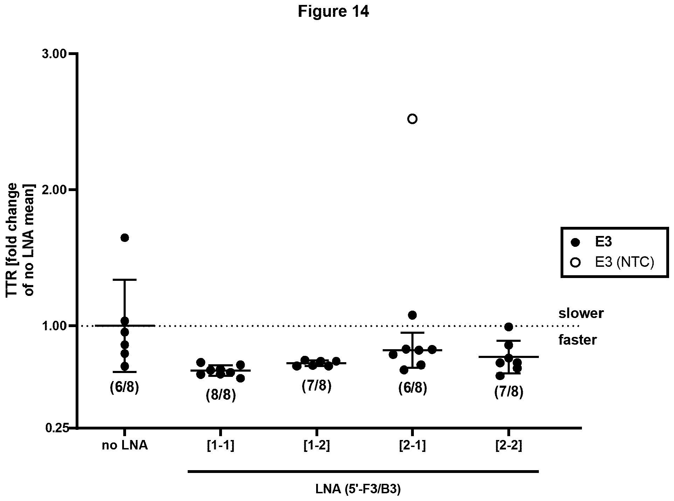

**Figure 14** shows that multiple combinations of different modified F3/B3 primers all lead to LAMP amplification improvements as shown by faster time-to-reaction (TTR), reduced amplification variance (standard deviation, SD), and in some cases enhanced sensitivity. RT-LAMP was used to amplify a region in the SARS-CoV-2 E gene. Base sequence of tested F3 and B3 primers was identical; only the position/number of LNA substitutions was different. E3, SARS-CoV-2 Envelope gene primer set 3.

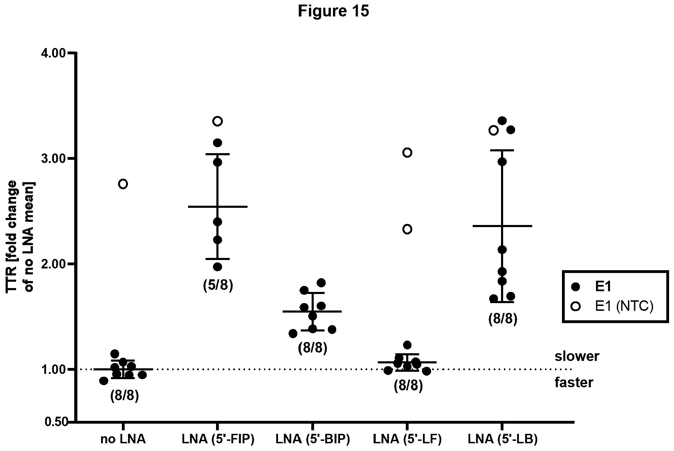

**Figure 15** shows that F3/B3 primers are the only ones which react positively to the introduction of LNA modifications. FIP, BIP, LF and LB primers were modified analogously to F3/B3 primers and these modified sets were used to amplify parts of the SARS-CoV-2 E gene. All tested set variants displayed deterioration of assay performance as demonstrated by increased variance (standard deviation, SD), slower time-to-reaction (TTR), and nonspecific amplification.

### DETAILED DESCRIPTION OF THE INVENTION

The present invention is described in detail in the following and is also illustrated by the appended examples and figures.

LAMP as such is e.g., already described in EP 1020534 B2.

However, prior art has failed to show a successful application of locked nucleic acid (LNA) primers in LAMP. The inventors, however, surprisingly found that the use of locked nucleic acids (LNA) in the F3/B3 primer further improves LAMP. As shown in Example 5, the use of LNAs in the F3/B3 primers reduces reaction times and improves reproducibility and sensitivity. Interestingly, this effect is only present when the LNA are located within the first third of the F3 or B3 primer.

Accordingly, the present invention relates to a LAMP method, comprising synthesizing a nucleic acid sequence from a template nucleic acid sequence in a reaction mixture comprising
(i) a first inner primer (FIP) nucleotide sequence and a second inner primer (BIP) nucleotide sequence,
(ii) a first outer primer (F3) nucleotide sequence and a second outer primer (B3) nucleotide sequence, wherein said F3 nucleotide sequence and/or said B3 nucleotide sequence comprises one or more locked nucleic acid (LNA) nucleotides which are located within the first third of said F3 nucleotide sequence or said B3 nucleotide sequence, respectively, wherein said first third is the first third of a nucleotide sequence in 5'- to 3'-orientation,
(iii) a DNA polymerase catalyzing a strand-displacement-type reaction of synthesizing a complementary nucleic acid strand from said template nucleic acid, and
(iv) nucleotides serving as substrates for said DNA polymerase at such a temperature that at least FIP nucleotide sequence and BIP nucleotide sequence can form stable base pairing with its complementary nucleotide sequences comprised by said template nucleic acid while the DNA polymerase activity can be maintained, thereby synthesizing a nucleic acid sequence.

In one embodiment, said F3 nucleotide sequence comprises one or more LNA nucleotides. In a further embodiment, said B3 nucleotide sequence comprises one or more LNA nucleotides. In a further embodiment, said F3 nucleotide sequence and said B3 nucleotide sequence comprise one or more LNA nucleotides.

"Loop mediated isothermal amplification" (LAMP) as used herein relates to a (single-tube) technique for the amplification of nucleic acids. Reverse Transcription Loop-mediated Isothermal Amplification (RT-LAMP) combines LAMP with (concurrent) reverse transcription to allow the detection of RNA. LAMP is an isothermal nucleic acid amplification technique. In contrast to the polymerase chain reaction (PCR) technology, in which the reaction is carried out with a series of alternating temperature steps or cycles, isothermal amplification is carried out at a constant temperature, and does not require a thermal cycler. LAMP is known to a person skilled in the art. In this context, we exemplarily refer to EP 1 020 534 B2, which describes LAMP in detail. In particular, we refer to paragraphs [0038] to [0054], including the figures cited in said paragraphs of EP 1 020 534 B2.

A locked nucleic acid (LNA), also known as bridged nucleic acid (BNA), and often referred to as inaccessible RNA, as used herein may relate to a modified RNA or DNA, preferably DNA, nucleotide in which the ribose moiety is modified with an extra bridge connecting the 2' oxygen and 4' carbon. The bridge "locks" the ribose in the 3'-endo (North) conformation. The bridge may be a methyl group covalently bound to the 2' oxygen and 4' oxygen, thereby bridging both oxygen atoms. LNAs are commercially available and a method of their synthesis is exemplarily described in Obika et al. (1997), Tetrahedron Letters, 38(50):8735-8738. LNA-modified bases include adenine (+A), cytosine (+C), guanine (+G), thymine (+T), and uracil (+U), preferably +A and +T. Preferably, said F3 nucleotide sequence comprises 1-5, preferably 3 LNA nucleotides. Preferably, said B3 nucleotide sequence comprises 1-5, preferably 3 LNA nucleotides.

The nucleic acid synthesized by the LAMP method of the invention may result in a nucleic acid having complementary nucleotide sequences linked alternately in a single-stranded chain. Such a nucleic acid may have mutually complementary nucleotide sequences linked side by side in a single-stranded chain. Further, in the present invention, it may contain a nucleotide sequence for forming a loop between the complementary chains. In the present invention, this sequence is called the loop-forming sequence. The nucleic acid synthesized by the present invention is composed substantially of mutually complementary chains linked via the loop-forming sequence. In general, a strand not separated into 2 or more molecules upon dissociation of base pairing is called a single-stranded chain regardless of whether it partially involves base pairing or not. The complementary nucleotide sequence can form base pairing in the same chain. An intramolecular base-paired product, which can be obtained by permitting the nucleic acid having complementary nucleotide sequences linked alternately in a single-stranded chain according to the present invention to be base-paired in the same chain, gives a region constituting an apparently double-stranded chain and a loop not involving base pairing.

That is, the nucleic acid having complementary nucleotide sequences linked alternately in a single-stranded chain according to the present invention contains complementary nucleotide sequences capable of annealing in the same chain, and its annealed product can be defined as single-stranded nucleic acid constituting a loop not involving base pairing at a bent hinged portion. A nucleotide having a nucleotide sequence complementary thereto can anneal to the loop not involving base pairing. The loop-forming sequence can be an arbitrary nucleotide sequence. The loop-forming sequence is capable of base pairing so as to initiate the synthesis of a complementary chain for displacement and is provided preferably with a sequence distinguishable from a nucleotide sequence located in the other region in order to achieve specific annealing. For example, in the present invention, the loop-forming sequence contains substantially the same nucleotide sequence as a region F2c (or B2c) located at the 3'-side of a region (i.e. F1c or B1c) derived from nucleic acid as a template and annealed in the same chain.

In the present invention, substantially the same nucleotide sequence is defined as follows. That is, when a complementary chain synthesized with a certain sequence as a template anneals to a target nucleotide sequence to give the origin of synthesizing a complementary chain, this certain sequence is substantially the same as the target nucleotide sequence. For example, substantially the same sequence as F2 includes not only absolutely the same nucleotide sequence as F2 but also a nucleotide sequence capable of functioning as a template giving a nucleotide sequence capable of annealing to F2 and acting as the origin of synthesizing complementary chain. The term "anneal" in the present invention means formation of a double-stranded structure of nucleic acid through base pairing based on the rules of Watson-Crick base pairing. Accordingly, even if a nucleic acid chain constituting base pairing is a single-stranded chain, annealing occurs if intramolecular complementary nucleotide sequences are base-paired. In the present invention, annealing and hybridization have the same meaning in that the nucleic acid constitutes a double-stranded structure through base pairing.

The number of pairs of complementary nucleotide sequences constituting the nucleic acid according to the present invention is at least 1. According to a desired mode of the present invention, it may be 2 or more. In this case, there is theoretically no upper limit to the number of pairs of complementary nucleotide sequences constituting the nucleic acid. When the nucleic acid as the synthetic product of the present invention is constituted of plural sets of complementary nucleotide sequences, this nucleic acid is composed of repeated identical nucleotide sequences.

The nucleic acid (having complementary nucleotide sequences linked alternately in a single-stranded chain) synthesized by the present invention may not have the same structure as naturally occurring nucleic acid. It is known that if a nucleotide derivative is used as a substrate when nucleic acid is synthesized by the action of a DNA polymerase, a nucleic acid derivative can be synthesized. The nucleotide derivative used may include nucleotides labeled with a radioisotope or nucleotide derivatives labeled with a binding ligand such as biotin or digoxigenin. These nucleotide derivatives can be used to label nucleic acid derivatives as the product. Alternatively, if fluorescent nucleotides are used as a substrate, the nucleic acid as the product can be a fluorescent derivative. Further, this product may be either DNA or RNA. Which one is formed is determined by a combination of the structure of a primer, the type of substrate for polymerization, and the composition of polymerization reagents for carrying out polymerization of a nucleic acid.

Synthesis of the nucleic acid having the structure described above can be initiated by use of a DNA polymerase having strand displacement activity and nucleic acid which is provided at the 3'-terminal thereof with a region F1 capable of annealing to a part F1c in the same chain and which upon annealing of the region F1 to F1c, is capable of forming a loop containing a region F2c capable of base pairing. There are many reports on the reaction of synthesizing complementary chain wherein a hairpin loop is formed and a sample sequence itself is used as a template, while in the present invention the portion of the hairpin loop is provided with a region capable of base pairing, and there is a novel feature on utilization of this region in synthesizing complementary chain. By use of this region as the origin of synthesis, a complementary chain previously synthesized with a sample sequence itself as a template is displaced. Then, a region B1c (arbitrary region) located at the 3'-terminal of the displaced chain is in a state ready for base-pairing. A region having a complementary sequence to this B1c is annealed thereto, resulting in formation of the nucleic acid (2 molecules) having a nucleotide sequence extending from F1 to B1c and its complementary chain linked alternately via the loop-forming sequence. In the present invention, the arbitrary region such as B1c above can be selected arbitrarily provided that it can be annealed to a polynucleotide having a nucleotide sequence complementary to that region, and that a complementary chain synthesized with the polynucleotide as the origin of synthesis has necessary functions for the present invention.

In the present invention, the term "nucleic acid" is used. The nucleic acid in the present invention generally includes both DNA and RNA. However, nucleic acid whose nucleotide is replaced by an artificial derivative or modified nucleic acid from natural DNA or RNA is also included insofar as it functions as a template for synthesis of complementary chain. The nucleic acid may be contained in a biological sample. The biological sample includes animal, plant or microbial tissues, cells, cultures and excretions, or extracts therefrom. The biological sample may include intracellular parasitic genomic DNA or RNA such as virus or mycoplasma. An exemplary virus may be SAR-CoV-2. The nucleic acid may be derived from nucleic acid contained in said biological sample. For example, cDNA synthesized from mRNA, or nucleic acid amplified on the basis of nucleic acid derived from the biological sample, is a typical example of the nucleic acid. The nucleic acid may be DNA

The term "template" used in the present invention may relate to a nucleic acid serving as a template for synthesizing a complementary chain. A complementary chain having a nucleotide sequence complementary to the template relates to a chain corresponding to the template, but the relationship between the two is merely relative. That is, a chain synthesized as the complementary chain can function again as a template. That is, the complementary chain can become a template. Preferably, the template nucleic acid sequence is single stranded or double stranded. Preferably, the template nucleic acid sequence is single- or double-stranded DNA, RNA or a DNA/RNA chimera. In the context of RT-LAMP, the template preferably is RNA.

Preferably, said FIP nucleotide sequence comprises at least two regions F2 and F1c, wherein said F1c region is linked to the 5'-side of the F2 region, wherein said F2 region has a nucleotide sequence complementary to an arbitrary region F2c in said template nucleic acid sequence, and wherein said F1c region has substantially the same nucleotide sequence as a region F1c located at the 5'-side of said F2c region in said template nucleic acid sequence.

Preferably, said BIP nucleotide sequence comprises at least two regions B2 and B1c, wherein said B1c region is linked to the 5'-side of the B2 region, wherein said B2 region has a nucleotide sequence complementary to an arbitrary region B2c in said template nucleic acid sequence strand, and wherein said B1c region has substantially the same nucleotide sequence as a region B1c located at the 5'-side of said region B2c in said template nucleic acid sequence.

Preferably, said F3 nucleotide sequence has a nucleotide sequence substantially complementary to a region F3c in said template nucleic acid sequence, wherein region F3c is located at the 3'-side of region F2c in said template nucleic acid sequence, wherein region F2c is located at the 3'-side of region F1c in said template nucleic acid sequence.

Preferably, said B3 nucleotide sequence has a nucleotide sequence substantially complementary to a region B3c in the template nucleic acid sequence, wherein region B3c is located at the 3'-side of region B2c in said template nucleic acid sequence, wherein region B2c is located at the 3'-side of region B1c in said template nucleic acid sequence.

Preferably, said reaction mixture further comprises loop primer nucleotide sequence F and/or loop primer nucleotide sequence B. Nagamine et al. (2002), Mol. Cell. Probes 16(3): 223-229, describe exemplary loop primer nucleotide sequences.

Preferably, said reaction mixture further comprises stem primer nucleotide sequence F and/or stem primer nucleotide sequence B. Gandelman et al. (2011), Int. J. Mol. Sci. 12: 9108-9124, describe exemplary stem primer nucleotide sequences.

Preferably, said reaction mixture further comprises swarm primer nucleotide sequence F1S and/or swarm primer nucleotide sequence B1S. Martineau et al. (2017), Anal. Chem. 89(1): 625-632 describe exemplary swarm primer nucleotide sequences.

Preferably, said reaction mixture further comprises at least a further first inner primer (FIP2) nucleotide sequence and a further second inner primer (BIP2) nucleotide sequence, both of which are different from said FIP and BIP nucleotide sequence. Wang et al. (2015), Molecules 20: 21515-21531 describe exemplary further first or second inner primer nucleotide sequences.

Preferably, said reaction mixture further comprises primer nucleotide sequences for performing said method as multiple cross displacement amplification LAMP. Wang et al. (2015), Sci Rep. 5: 11902, describe an exemplary multiple cross displacement amplification LAMP.

Preferably, said reaction mixture further comprises primer nucleotide sequences for performing said method as reverse transcription isothermal multiple self matching LAMP. Ding et al. (2014), J. Clin. Microbiol. 52(6): 1862-1870, disclose an exemplary reverse transcription isothermal multiple self matching LAMP.

The method of synthesizing nucleic acid according to the present invention is supported by the DNA polymerase catalyzing the strand displacement-type reaction for synthesis of complementary chain. It is advantageous to use one kind of DNA polymerase. Exemplary suitable polymerases are listed in the following. Further, various mutants of these enzymes can be utilized in the present invention insofar as they have both the sequence-dependent activity for synthesis of complementary chain and the strand displacement activity. The mutants referred to herein include those having only a structure bringing about the catalytic activity required of the enzyme or those with modifications to catalytic activity, stability or thermostability by, e.g., mutations in amino acids. Exemplary polymerases include, but are not limited to, *Bst* DNA polymerase, *Bst* 2.0 WarmStart^{®} DNA polymerase / *Bst* 3.0 DNA polymerase (New England Biolabs., Inc.), Saphir Bst2.0 polymerase (Jena Bioscience GmbH), GspSSD LF DNA Polymerase / GspSSD2.0 LF DNA Polymerase / GspM3.0 LF DNA Polymerase (OptiGene Ltd.), Bca (exo-)DNA polymerase, DNA polymerase I Klenow fragment, Vent DNA polymerase, Vent (exo-)DNA polymerase (Vent DNA polymerase deficient in exonuclease activity), Deep Vent DNA polymerase, Deep Vent(exo-)DNA polymerase (Deep Vent DNA polymerase deficient in exonuclease activity), Φ29 phage DNA polymerase, MS-2 phage DNA polymerase, Z-Taq DNA polymerase (Takara Shuzo Co., Ltd.), KOD DNA polymerase (Toyobo Co., Ltd.), OmniAmp^{®} and LavaLAMP^{™} DNA/RNA Enzyme (Lucigen Corp.), and SD Polymerase (BIORON GmbH).

Among these enzymes, GspSSD2.0 LF DNA Polymerase, LavaLAMP^{™} DNA/RNA Enzyme, SD Polymerase, and Bst DNA polymerase, more preferably *Bst* 2.0 WarmStart^{®} DNA polymerase are particularly desired enzymes because they have a certain degree of thermostability and high catalytic activity. The reaction of this invention can be carried isothermally in a preferred embodiment. Preferably, the enzyme is thermostable. Although the isothermal reaction is feasible, heat denaturation may be conducted to provide nucleic acid as a first template, and in this respect too, utilization of a thermostable enzyme broadens selection of assay protocol.

Vent (exo-)DNA polymerase is an enzyme having both strand displacement activity and a high degree of thermostability. It is known that the complementary chain synthetic reaction involving strand displacement by DNA polymerase is promoted by adding a single strand binding protein (Paul M. Lizardi et al., Nature Genetics, 19, 225-232, July, 1998). This action is applied to the present invention, and by adding the single strand binding protein, the effect of promoting the synthesis of complementary chain can be expected. For example, T4 gene 32 is effective as a single strand binding protein for Vent (exo-) DNA polymerase. Preferably, said DNA polymerase is a DNA-dependent DNA polymerase or an RNA-dependent DNA polymerase. Preferably, said DNA polymerase has reverse transcriptase activity.

Preferably, said F3 nucleotide sequence has 15-30, preferably 17-25 nucleotides in length.

Preferably, said B3 nucleotide sequence has 15-30, preferably 17-25 nucleotides in length.

The method LNA-LAMP method of the invention is preferably carried out in the presence of a buffer giving suitable pH to the enzyme reaction, salts necessary for annealing or for maintaining the catalytic activity of the enzyme, a protective agent for the enzyme, and as necessary a regulator for melting temperature (Tm). Exemplary buffers are known to a person skilled in the art and include, e.g., Tris-HCl or equivalents. The pH is adjusted depending on the DNA polymerase used. The buffer may be added at a concentration of 1 mM or more, such as more than 1 mM or 1.5mM or more. Exemplary salts such as KCI, NaCl, MgCl₂, (NH₄)₂SO₄ etc. are suitably added to maintain the activity of the enzyme and to regulate the melting temperature (Tm) of nucleic acid. Protective agents to maintain enzymatic activity include e.g., bovine serum albumin (BSA), reducing agents like tris(2-carboxyethyl)phosphine (TCEP), (2S,3S)-1,4-Bis(sulfanyl)butane-2,3-diol (DTT), and/or sugars. Further, dimethyl sulfoxide (DMSO) or formamide may be used as the regulator for melting temperature (Tm). By use of the regulator for melting temperature (Tm), annealing of the oligonucleotide can be regulated under limited temperature conditions. Further, betaine (N,N,N-trimethylglycine) or a tetraalkyl ammonium salt is also effective for improving the efficiency of strand displacement by virtue of its isostabilization. Betaine can be added at a concentration of 0.2 to 3.0 M, preferably 0.5 to 1.5 M to the reaction solution. Accordingly, the reaction mixture preferably further comprises a regulator of melting temperature, e.g., betaine, preferably in a concentration between 0.2 to 3.0 M, guanidine thiocyanate or hydrochloride in a concentration preferably between 20 and 80 mM, single-stranded binding protein (SSB), and/or tetramethyl ammonium chloride (TMAC) in a concentration preferably between 5 mM and 80 mM. In addition, the reaction mixture may comprise a stabilizer, e.g., BSA in a concentration preferably between 0.02 mg/ml and 2 mg/ml, a non-ionic surfactant such as Tween-20/Triton X-100 in a concentration preferably between 0.02 % and 0.2% v/v and/or TCEP/DTT preferably in a concentration between 0.5 mM to 5 mM.

The methods of the present invention may be used for the detection of a given template such as a nucleic acid derived from a pathogen such as SARS-CoV-2. Accordingly, said reaction mixture may further comprise a detector for detecting the product of the nucleic acid sequence synthesis reaction.

The detector (for detecting the product of the nucleic acid sequence synthesis reaction) may be a metallochromic indicator. Further examples of a detector are a DNA-intercalating substance or a pH-sensitive dye, with a metallochromic indicator being preferred. As described herein, a metallochromic indicator is capable of building a complex with metal ions, e.g., magnesium. As also described herein, preferred metal ions are transition metal ions or post-transition metal ions.

A metallochromic indicator or, as also referred to herein, a complexometric indicator, is a molecule which can be bound by metal ions, i.e., it builds a complex with metal ions. Such a complex is a metallochromic indicator-metal ion complex, or, in short, as referred to herein "complex". A complex has preferably a different colorimetric or fluorescence property than a non-complex/a metallochromic indicator not-complexed with metal ions, i.e., a metallochromic indicator in the absence of metal ions, i.e., not building a complex with metal ions, thus being in a non-complex or non-complexed. Accordingly, the detector preferably comprises a transition metal or post-transition metal. The detector preferably does not form or build a complex with magnesium.

Such a complex is reversible which means metal ions when bound to a metallochromic indictor can be/are released from the complex with the metallochromic indicator. A release of metal ions from the complex may occur, when a binding partner for the metal ions is at the same time present or will be added or generated, e.g., through a (chemical) reaction and which has a sufficient concentration and/or affinity for the metal ions bound by the metallochromic indicator.

A preferred metallochromic indicator provides in the context of the present invention, e.g., in the methods, uses or kits for the detection of a change of spectral or fluorescent properties of its complex with metal ions. Such a change occurs when metal ions become absent or are released from the complex. In the context of the present invention, during an amplification of a nucleic acid sequences, pyrophosphate occurs, which is a binding partner for a metal ion being in a complex with a metallochromic indicator. If pyrophosphate has a sufficient concentration and/or affinity for the metal ion bound by the metallochromic indicator, the metal ion is released from the complex and binds with/to pyrophosphate, thereby forming a metal ion-pyrophosphate salt.

A preferred change in the spectral properties of said complex is in the spectrum from 380 to 740 nm. Accordingly, the change in the spectral properties of said complex in its spectral properties is preferably a change of the color of the complex when compared to the metallochromic indicator not bound by metal ions, i.e., when metal ions no longer build a complex (with the metallochromic indicator) or are released from the complex. This may occur, as described above, since e.g., metal ions are released from the complex in the presence of, e.g., pyrophosphate which is generated during an amplification of a nucleic acid sequence as described herein.

In contrast to the present invention, the prior art applied for the detection of nucleic acid sequences metallochromic indicators, e.g., hydroxynaphtol blue (HNB) which build a complex the metal magnesium, i.e., a non-transition/non-post transition metal. Magnesium is an important ion for amplification methods (PCR, LAMP, etc.) as it serves as a cofactor and catalyst for enzyme activity and influences annealing of primers, stability of DNA, and incorporation of nucleotides. Because of this, using a colorimetric detection system that is dependent on Mg²⁺ ions is problematic as the levels of free Mg²⁺ for the enzyme and template vary during the course of the reaction. In addition, a colorimetric detection system that is dependent on Mg²⁺ prevents independent optimization of the detection system and the amplification reaction as both are tied to the concentration of the same ion. Accordingly, the detector or the metallochromic indicator preferably does not build a complex with magnesium. A detection system that uses a metal ion that is inert in the reaction is thus preferred. Suitable metal ions, in particular transition metal or post-transition metal ions, in the context of the invention may be Ba²⁺, Sr²⁺, Zn²⁺, Cd²⁺, Cu²⁺, Co²⁺, Ni²⁺, Hg²⁺, Pb²⁺, Pt²⁺, Ru²⁺, Rh²⁺, Fe²⁺, In³⁺, Al³⁺, Bi³⁺, La³⁺, Sc³⁺, Th³⁺, and/or Zr³⁺ ions, with Zn²⁺, Cu²⁺, Co²⁺, Ni²⁺, Pt²⁺, Ru²⁺, Rh²⁺, Fe²⁺ ions being preferred, with Cu²⁺, Co²⁺, Ni²⁺, Zn²⁺ and/or Fe²⁺ ions being preferred, with Zn²⁺ ions being more preferred.

Also in contrast to the present invention, the prior art applied for the detection of nucleic acid sequences metallochromic indicators, e.g., calcein which build a complex with the non-transition metal manganese. Such a complex, when releasing metal ions provides for a change in its fluorescent properties. Manganese, while not normally included in PCR/LAMP reaction buffer, is known to have dramatic effects on amplification and thus should be avoided. While Mn²⁺ has been found to substitute for Mg²⁺ in PCR, it is well known to reduce the fidelity of DNA polymerases and consequently induce random mutagenesis during PCR. At high concentrations (e.g., 500 µM and above), Mn²⁺ can also be inhitory to PCR - the calcein/Mn²⁺ detection system uses 1 mM Mn²⁺.

In the context of the present invention, a metallochromic indicator is used to bind to metal ions added to a reaction mixture comprising a template nucleic acid sequence, primer nucleotide sequences, nucleotides and a polymerase capable of amplifying the nucleic acid molecule. Of course, the metallochromic indicator and metal ions are also comprised by the reaction mixture. Accordingly, the metallochromic indicator binds metal ions to build a complex. Without being bound by theory, during the amplification of nucleotide sequences, more and more pyrophosphate is released. The increasing amount of pyrophosphate will then bind the metal ions bound by the metallochromic indicator. As a result, the metallochromic indicator will change its spectral and/or fluorescent properties. This change serves as the indicator that the amplification reaction (successfully) takes place or has taken place.

Preferably, a pyrophosphate metal ion complex or salt has a higher affinity for the metal ion than the metallochromic indicator. This means the equilibrium of the metal ions is preferably shifted to the pyrophosphate than to the metallochromic indicator. Or, preferably, a pyrophosphate metal ion complex or salt is at a sufficient concentration to shift the equilibrium of the metal ions to complexing with pyrophosphate rather than the metallochromic indicator. The skilled person can readily determine this.

Also, the skilled person can determine the amount of metallochromic indicator and/or metal ions when used in a reaction mixture as described herein such that the desired change in spectral or fluorescent properties of the complex between a metallochromic indicator and metal ions can be detected. A change occurs when metal ions are released from the complex between a metallochromic indicator and metal ions, because more and more pyrophosphate is generated during the amplification of nucleic acid sequences as described herein and is commonly known in the art that pyrophosphate is released if a polymerase incorporates a nucleotide into DNA or RNA.

Preferred metal ions used in the context of the present invention, e.g., in the context of the methods, uses or kits described herein are transition metal ions or post-transition metal ions. A "transition metal" as used herein is an element selected from the group consisting of Sc, Ti, V, Cr, Mn, Fe, Co, Ni, Cu, Y, Zr, Nb, Mo, Tc, Ru, Rh, Pd, Ag, La, Hf, Ta, W, Re, Os, Ir, Pt, Au, Th, Rf, Db, Sg, Bh and Hs. Sr and Ba may be seen as being further elements of the transition metal group. A "post-transition metal" as used herein is an element selected from the group consisting of Al, Zn, Ga, Cd, In, Sn, Hg, TI, Pb, Bi, Po and At. Preferred examples of metal ions in general and of transition or post-transition metal ions in particular are Ba²⁺, Sr²⁺, Zn²⁺, Cd²⁺, Cu²⁺, Co²⁺, Ni²⁺, Hg²⁺, Pb²⁺, Pt²⁺, Ru²⁺, Rh²⁺, Fe²⁺, In³⁺, Al³⁺, Bi³⁺, La³⁺, Sc³⁺, Th³⁺, Zr³⁺ ions, with Zn²⁺, Cu²⁺, Co²⁺, Ni²⁺, Pt²⁺, Ru²⁺, Rh²⁺, Fe²⁺ ions being preferred, with Cu²⁺, Co²⁺, Ni²⁺, Zn²⁺ and Fe²⁺ ions being preferred, with Zn²⁺ ions being more preferred.

Preferred examples of metallochromic indicators are those which are able to bind metal ions, preferably transition metal ions or post-transition metal ions. Examples of preferred metallochromic indicators are pyrocatechol violet, dithizone, zincon, eriochrome black T, murexide, PAN, phthalein purple, xylenol orange, 2-(5-Bromo-2-pyridylazo)-5-[N-propyl-N-(3-sulfopropyl)amino]phenol (5-Br-PAPS). Examples of more preferred metallochromic indicators are pyrocatechol violet, zincon, dithizone, PAN, 5-Br-PAPS. Examples of even more preferred metallochromic indicators are zincon and 5-Br-PAPS. 5-Br-PAPS is most preferred.

The structures of the more preferred metallochromic indicators zincon as well as zincon derivatives are depicted below. In the case of zincon derivatives, R₁, R₂, and R₃ can represent one or more functional groups attached to the associated phenyl rings:

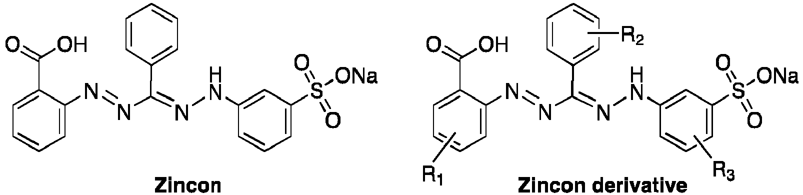

The structures of the more preferred metallochromic indicators PAN and PAR and PAN/PAR derivatives are depicted below. In the case of PAN/PAR derivatives, R can represent one or more functional groups connected to the phenol ring:

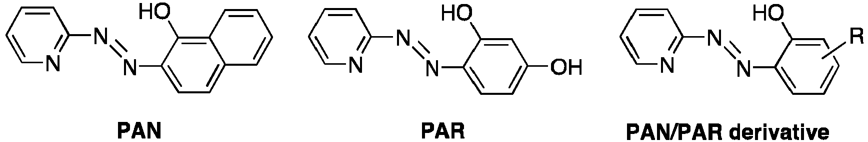

The term "5-Br-PAPS" also encompasses derivatives of 5-Br-PAPS which are also preferred. Such derivatives may have one or more modifications, but are still capable of forming a complex with metal ions, and having identical, but at least similar spectral properties as 5-Br-PAPS when bound to metal ions. Exemplary 5-Br-PAPS (X = Br) derivatives are 2-(5-Fluoro-2-pyridylazo)-5-[N-propyl-N-(3-sulfopropyl)amino]phenol (X = F), 2-(5-Chloro-2-pyridylazo)-5-[N- 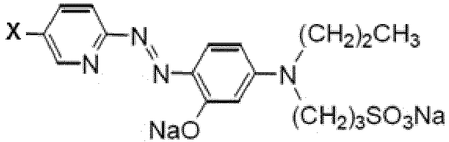 propyl-N-(3-sulfopropyl)amino]phenol (X = CI), 2-(5-lodo-2-pyridylazo)-5-[N-propyl-N-(3-sulfopropyl)amino]phenol (X = I), 2-(5-Nitro-2-pyridylazo)-5-[N-propyl-N-(3-sulfopropyl)amino]phenol (X = NO₂), or 2-(5-Cyano-2-pyridylazo)-5-[N-propyl-N-(3-sulfopropyl)amino]phenol (X = CN). The following structure shows a PAPS backbone in form of its disodium salt dehydrate, in which X can be replaced as described above:

As illustrated in the structure below, X can be replaced as described above and can also occupy alternative positions in the pyridine ring (e.g., positions 3-6) as a PAPS derivative:

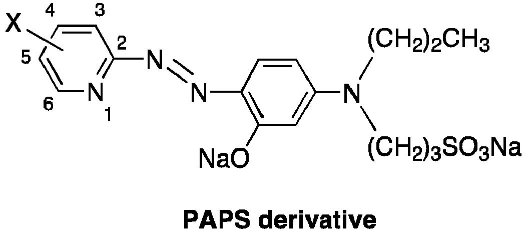

The following Table 1 shows some further exemplary metallochromic indicators and its change in spectral properties with and without metal ions.

**Table 1. Exemplary metallochromic indicators and their corresponding changes in spectral properties with and without metal ions.**

| **Indicator** | **Color with metal ion** | **Color without metal ion** |
|---|---|---|
| 5-Br-PAPS | magenta or blue | orange-yellow |
| Dithizone | red | green-violet |
| Eriochrome Black T | purple-red | blue |
| Murexide | yellow | violet |
| PAN | red | yellow |
| Phtalein purple | purple | colorless |
| Pyrocatehcol violet | blue | yellow |
| Xylenol orange | red | yellow |
| Zincon | blue | orange-yellow |

The following Table 2 shows more preferred metallochromic indicators, visible and spectral properties, with and without preferred metal ions in the presence of magnesium ions.

**Table 2. More preferred metallochromic indicators, visible and spectral properties, with and without preferred metal ions in the presence of magnesium ions.**

| | **Complex color in the presence of Mg²⁺** | | | | | | |
|---|---|---|---|---|---|---|---|
| **Indicator** | **No metal ion** | **Co²⁺** | **Cu²⁺** | **Fe²⁺** | **Ni²⁺** | **Rh²⁺** | **Zn²⁺** |
| 5-Br-PAPS | Yellow-orange | Violet | Purple | Purple | Magenta | Dark Green | Magenta |
| PAR | Yellow-green | Red | Orange | Light Orange | Orange | Brown | Orange |
| Zincon | Orange-red | Green | Blue | Light Brown | Brown | Orange-red | Dark Blue |

In one embodiment, e.g., of the LAMP method of the invention, the detector is a metallochromic indicator, preferably 2-(5-Bromo-2-pyridylazo)-5-[N-propyl-N-(3-sulfopropyl)amino]phenol (5-Br-PAPS) in combination with a metal ion, preferably a transition metal or post-transition metal, more preferably Zn²⁺, Cu²⁺, Co²⁺, Ni²⁺, Pt²⁺, Ru²⁺, Rh²⁺, and/or Fe²⁺ ions, most preferably Zn²⁺ ions, said metallochromic indicator and each of said metal ions building a complex, but not with magnesium, and wherein the method further comprises (b) detecting a change in spectral or fluorescent properties of the complex resulting from the amplification of said nucleic acid sequence.

In one embodiment, of the LAMP method of the invention, the detector is 5-Br-PAPS in combination with metal ions. Metal ions in this context may include any transition or post-transition metal that is chelated by both 5-Br-PAPS and pyrophosphate, including, but not limited to, Zn²⁺, Cu²⁺, Co²⁺, Ni²⁺, Pt²⁺, Ru²⁺, Rh²⁺, and/or Fe²⁺ ions, preferably Zn²⁺ ions. Accordingly, the reaction mixture, used in the methods of the invention, may further comprise 2-(5-Bromo-2-pyridylazo)-5-[N-propyl-N-(3-sulfopropyl)amino]phenol (5-Br-PAPS) and Zn²⁺, Cu²⁺, Co²⁺, Ni²⁺, Pt²⁺, Ru²⁺, Rh²⁺, and/or Fe²⁺ ions, preferably Zn²⁺ ions, said 5-Br-PAPS and each of said ions building a complex. In this context, the method of the invention further comprises (b) detecting a change in spectral or fluorescent properties of the complex resulting from the amplification of said nucleic acid sequence.

5-Br-PAPS is a compound that has the ability to bind transition or post-transition metals including, but not limited to, Zn²⁺, Cu²⁺, Co²⁺, Ni²⁺, Pt²⁺, Ru²⁺, Rh²⁺, and/or Fe²⁺ ions with high affinity, thereby building a complex, leading to a color change or in other words, a change in spectral or fluorescent properties. If the reaction mixture contains Zn²⁺, Cu²⁺, Co²⁺, Ni²⁺, Pt²⁺, Ru²⁺, Rh²⁺, and/or Fe²⁺ ions, preferably Cu²⁺, Co²⁺, Ni²⁺, Zn²⁺ and Fe²⁺ ions, more preferably Zn²⁺ ions, the reaction mixture is colored In case the metal ion is Zn²⁺, the reaction mixture is colored magenta (see also Fig. 1). Without being bound by theory, the synthesizing reaction of the LNA-LAMP method of the invention or any other nucleic acid-yielding polymerase (chain) reaction leads to a release of pyrophosphate (PPᵢ) from the triphosphorylated nucleotides (e.g., ATP, GTP, CTP, TTP, UTP etc.) during the elongation reaction. Notably, PPᵢ is also a chelator for metal ions such as transition and/or post-transition metals, including, but not limited to, Zn²⁺, Cu²⁺, Co²⁺, Ni²⁺, Pt²⁺, Ru²⁺, Rh²⁺, and/or Fe²⁺ ions, preferably Cu²⁺, Co²⁺, Ni²⁺, Zn²⁺ and Fe²⁺ ions, more preferably Zn²⁺ ions, that are capable of breaking the complex between 5-Br-PAPS and Zn²⁺, Cu²⁺, Co²⁺, Ni²⁺, Pt²⁺, Ru²⁺, Rh²⁺, and/or Fe²⁺ ions, preferably Cu²⁺, Co²⁺, Ni²⁺, Zn²⁺ and Fe²⁺ ions, more preferably Zn²⁺ ions, thereby leading to a "color transition" of the reaction mixture from magenta to orange-yellow (see examples 1-4) and changing the spectral or fluorescent properties of the complex. Thus, in case the template to be verified is present, the elongation reaction takes place leading to release of PPᵢ. The PPᵢ in turn complexes the Zn²⁺, Cu²⁺, Co²⁺, Ni²⁺, Pt²⁺, Ru²⁺, Rh²⁺, and/or Fe²⁺ ions, preferably Cu²⁺, Co²⁺, Ni²⁺, Zn²⁺ and Fe²⁺ ions, more preferably Zn²⁺ ions, that were complexed by 5-Br-PAPS initially. The corresponding color change in spectral or fluorescent properties can be used as an indicator for the presence of the template. Specifically, increasing PPi concentrations weaken the 560 nm (± 10 nm) absorption peak ("magenta") of the reaction mixture with a new 448 nm (± 10 nm) peak ("orange-yellow") growing simultaneously, in particular if the metal ion is Zn²⁺. This color transition can be monitored in real-time.

In this context, the present invention further relates to an *in vitro* method for detecting a nucleic acid sequence amplification product, comprising
(a) contacting a reaction mixture comprising a template nucleic acid sequence, primer nucleotide sequences, nucleotides and a polymerase capable of amplifying said nucleic acid molecule with a metallochromic indicator and a transition or post-transition metal ion, said metallochromic indicator and said ion building a complex, but not building a complex with magnesium;
(b) amplifying said nucleic acid sequence under suitable conditions to obtain a nucleic acid sequence amplification product;
(c) detecting a change in spectral or fluorescent properties of the complex resulting from the amplification of said nucleic acid sequence, wherein formation of said complex comprising said metallochromic indicator and said transition or post-transition metal ion leads to a color change of the solution; wherein the amplification is performed by the LAMP method of the present invention.

Preferably, the metallochromic indicator is 5-Br-PAPS. The metal ion is a transition metal ion or post-transition metal ion, preferably a Zn²⁺ ion. Preferably, the change in spectral properties of said complex is in the spectrum from 380 to 740 nm wavelength. More preferably, the spectral change can be monitored at 560 nm (± 10 nm) ("magenta") and/or 448 nm (± 10 nm) ("orange-yellow"). In other words, a change in spectral properties of the reaction mixture from magenta to orange-yellow is visible, if a nucleic acid amplification product is produced in the *in vitro* method for detecting a nucleic acid sequence amplification product or other methods, uses and kits described herein.

Importantly, the change in spectral or fluorescent properties of the (5-Br-PAPS) complex is not dependent on a pH change of the reaction mixture (see Example 4, Fig. 7 and 8). The change in spectral or fluorescent properties is only dependent on the availability of metal ions such as Zn²⁺, Cu²⁺, Co²⁺, Ni²⁺, Pt²⁺, Ru²⁺, Rh²⁺, and/or Fe²⁺ ions, preferably Zn²⁺ ions. The availability in turn is dependent on the amount of PPᵢ in the reaction mixture but preferably not on the pH. This is in contrast to methods described in prior art, e.g., those as shown in EP2888374B1. Consequently, the reaction mixture may comprise a 1 mM or more of Tris (Tris(hydroxymethyl)aminomethane) buffer or equivalent buffer such as more than 1 mM, more than 1.5 mM, more than 5 mM, more than 10 mM, more than 50 mM, more than 100 mM or more than 250 mM Tris buffer or equivalent buffer. In this context, also a pH-sensitive polymerase may be used.

Step (b) of the *in vitro* method for detecting a (template) nucleic acid sequence amplification product is done by carrying out the LNA-LAMP method of the present invention.

The detection of changes in the spectral properties of the (5-Br-PAPS) complex can be achieved by their photochemical properties using for example, the eyes of the operator, a fluorimeter, or a spectrophotometer. The term "detecting" may be used interchangeably with the term "monitoring".

The final molar ratio of metallochromic indicator, preferably 5-Br-PAPS, to the metal ion, preferably Zn²⁺, Cu²⁺, Co²⁺, Ni²⁺, Pt²⁺, Ru²⁺, Rh²⁺, and/or Fe²⁺ ions, preferably Zn²⁺ ions, may be 2:1. This corresponds to the stoichiometry of the Ion²⁺/5-Br-PAPS complex in aqueous solutions. The Ion²⁺/5-Br-PAPS complex may be added to a final concentration of about 1 to about 250 µM, about 10 to about 100 µM, about 15 to about 75 µM, about 25 to about 75 µM, about 25 µM or about 50 µM.

The LAMP method of the present invention may be used for different purposes. In one embodiment, the LAMP method of the invention is for synthesizing a nucleic acid sequence. In one embodiment, the LAMP method of the invention is for detecting a nucleic acid sequence. In one embodiment, the LAMP method of the invention is for diagnosing a disease, preferably caused by a pathogen.

The present invention further relates to a kit for the synthesis of a nucleic acid sequence by loop mediated isothermal amplification (LAMP) on a template nucleic acid sequence, comprising
(i) a first outer primer (F3) nucleotide sequence and a second outer primer (B3) nucleotide sequence, each of which comprises locked nucleic acid (LNA) nucleotides, which are located within the first third of said F3 nucleotide sequence or said B3 nucleotide sequence, respectively, wherein said first third is the first third of a nucleotide sequence in 5'- to 3'-orientation,
(ii) a first inner primer (FIP) nucleotide sequence and a second inner primer (BIP) nucleotide sequence,
(iii) a DNA polymerase catalyzing a strand-displacement-type reaction of synthesizing a complementary nucleic acid strand from said template nucleic acid,
(iv) nucleotides serving as substrates for said DNA polymerase.

The F3 nucleotide sequence and/or said B3 nucleotide sequence may comprise one or more locked nucleic acid (LNA) nucleotides which are located within the first third of said F3 nucleotide sequence or said B3 nucleotide sequence. The F3 nucleotide sequence may comprise one or more locked nucleic acid (LNA) nucleotides which are located within the first third of said F3 nucleotide sequence. The B3 nucleotide sequence may comprise one or more locked nucleic acid (LNA) nucleotides which are located within the first third of said B3 nucleotide sequence. "First third" as used herein relates to the first third of a nucleotide sequence in 5'- to 3'-orientation, i.e., the nucleotides of the third of a nucleotide sequence, which is at the 5'-end of the nucleotide sequence.

5-Br-PAPS and Zn²⁺, Cu²⁺, Co²⁺, Ni²⁺, Pt²⁺, Ru²⁺, Rh²⁺, and/or Fe²⁺ ions, preferably Zn²⁺ ions, are further useful components of a kit since they will enable to carry out the detection of the presence of the template nucleic acid sequence. Accordingly, the kit of the invention may further comprise (v) 2-(5-Bromo-2-pyridylazo)-5-[N-propyl-N-(3-sulfopropyl)amino]phenol (5-Br-PAPS); and optionally a transition or post-transition metal that includes, but is not limited to, (vi) Zn²⁺, Cu²⁺, Co²⁺, Pt²⁺, Ru²⁺, Rh²⁺, and/or Fe²⁺ ions, preferably Zn²⁺ ions. The kit of the invention may further comprise a metallochromic indicator, preferably 5-Br-PAPS; and/or metal ions, preferably Zn²⁺ ions.

The present disclosure further relates to an aqueous preparation comprising: 5-Br-PAPS, a (DNA) polymerase, dNTPs, in a formulation that contains a buffering agent in an amount of 1 mM or more Tris or equivalent buffering agent such as more than 1 mM, more than 1.5 mM, more than 5 mM, more than 10 mM, more than 50 mM, more than 100 mM or more than 250 mM Tris buffer or equivalent buffer. The aqueous preparation may further comprise primers, preferably the primers are F3 nucleotide sequence and/or said B3 nucleotide sequence as defined herein. The aqueous preparation may further comprise nucleotides serving as substrates for said DNA polymerase. The aqueous preparation may further comprise Zn²⁺, Cu²⁺, Co²⁺, Ni²⁺, Pt²⁺, Ru²⁺, Rh²⁺, and/or Fe²⁺ ions, preferably Zn²⁺ ions.

The present disclosure further relates to an aqueous preparation comprising a first outer primer (F3) nucleotide sequence and a second outer primer (B3) nucleotide sequence as defined herein. The aqueous preparation may further comprise a first inner primer (FIP) nucleotide sequence and a second inner primer (BIP) nucleotide sequence as defined herein. The aqueous preparation may further comprise a DNA polymerase catalyzing a strand-displacement-type reaction of synthesizing a complementary nucleic acid strand from said template nucleic acid. The aqueous preparation may further comprise nucleotides serving as substrates for said DNA polymerase.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g., more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. When used herein the term "comprising" can be substituted with the term "containing" or "including" or sometimes when used herein with the term "having". When used herein "consisting of" excludes any element, step, or ingredient not specified.

The term "including" means "including but not limited to". "including" and "including but not limited to" are used interchangeably.

It should be understood that this invention is not limited to the particular methodology, protocols, material, reagents, and substances, etc., described herein and as such can vary. The terminology used herein is for the purpose of describing particular embodiments only and is not intended to limit the scope of the present invention, which is defined solely by the claims.

Nothing herein is to be construed as an admission that the invention is not entitled to antedate publications cited herein by virtue of prior invention. To the extent that the disclosure of publications cited herein contradicts or is inconsistent with this specification, the specification will supersede any such material.

### EXAMPLES

An even better understanding of the present invention and of its advantages will be evident from the following examples, offered for illustrative purposes only. The examples are not intended to limit the scope of the present invention in any way.

### Example 1: Comparison of preferred detection reagents

Our preferred detection reagent was selected in an initial screen to identify chelatometric dyes with desired spectral characteristics, namely ability to form colored complexes with preferred metal ions in the presence of magnesium, lucidness of observed color change and high absorbance of the formed dye-metal complex. Combinations of potential Mg²⁺-insensitive detection reagents were prepared under buffered conditions (20 mM Tris-HCl pH=7.9, 50 mM KCI) in the presence of high magnesium concentrations (8 mM MgSO₄) by mixing one of the preferred complexometric dyes (5-Br-PAPS, PAR or zincon; all sourced from Sigma-Aldrich, Missouri, USA) with one of the preferred metal ions (Co²⁺, Cu²⁺, Fe²⁺, Ni²⁺, Rh²⁺, Zn²⁺) in such a way that the final concentration of complexometric dyes was 100 µM while the concentration of metal ions was either 50 µM (with 5-Br-PAPS, PAR) or 100 µM (with zincon) reflecting the predicted stoichiometry of the formed dye-metal complexes.

Spectral analysis on a Tecan Infinite M1000 Pro Multi-mode Reader (Tecan, Switzerland) was used to determine the λₘₐₓ of free dyes and their dye-metal complexes while pictures were taken with a cell phone camera. As can be seen in Fig 1A-B, all combinations with the exception of Zincon/Rh²⁺ exhibited dye-metal complex formation in the presence of high concentrations of magnesium demonstrating their potential to be used for colorimetric detection in the presence of magnesium. However as shown in Fig 1C, 5-Br-PAPS complexes had much higher average absorbance values than complexes of PAR and zincon which translated into more lucid color shifts between free and complexed forms of the dye. For this reason, 5-Br-PAPS was chosen as the most preferred chelatometric dye as higher absorbance values necessitate lower concentrations of both the dye and the metal, both of which can interfere with enzymatic activity in an assay.

5-Br-PAPS/metal complexes were further compared with respect to their ability to release chelated metal ions in the presence of pyrophosphate (PPi). 100 µM complexes of 5-Br-PAPS and preferred metal ions (Co²⁺, Cu²⁺, Fe²⁺, Ni²⁺, Rh²⁺, Zn²⁺, 50 µM each) were exposed to progressively higher concentrations of PPi (from 0 to 20 mM) and absorbances at dye-metal complex λₘₐₓ were measured spectrophotometrically on a Tecan Infinite M1000 Pro Multi-mode Reader (Tecan, Switzerland). Results (Fig 2A-B) demonstrated that while all 5-Br-PAPS/metal complexes reacted with PPi to release the complexed metal from 5-Br-PAPS, the Zn²⁺/5-Br-PAPS complex was by far the most easily disrupted by PPi (near complete complex disruption was observed at the lowest tested concentration). As this should lead to the highest sensitivity in a PPi producing assay, the Zn²⁺/5-Br-PAPS complex was chosen as our most preferred detection reagent which was used in further testing.

### Example 2: Characterization of the Zn²⁺/5-Br-PAPS detection reagent

To prepare the most preferred Zn²⁺/5-Br-PAPS detection reagent, a solution of 5-Br-PAPS is mixed with a solution of ZnSO₄ (both in PCR-grade water) in such a way, that the final molar ratio of 5-Br-PAPS to ZnSO₄ is 2:1 corresponding to the stoichiometry of the Zn²⁺/5-Br-PAPS complex in aqueous solutions. A 1.5 mM solution of the Zn²⁺/5-Br-PAPS detection reagent (1.5 mM 5-Br-PAPS, 750 µM ZnSO₄) is stable at room temperature in the dark and can be used to prepare reaction mixes.

The Zn²⁺/5-Br-PAPS detection reagent changes color during DNA amplification due to PPi (produced as a side-product of dNTP hydrolysis by a polymerase) competing with 5-Br-PAPS to bind Zn²⁺ (Fig. 3A). As amplification progresses, PPi accumulates and the increasing concentration of PPi progressively shifts the Zn²⁺/5-Br-PAPS complex (magenta) to free 5-Br-PAPS (orange-yellow) and zinc(ll) pyrophosphate. This mechanism was readily demonstrated under target assay conditions (maximum PPi production ~5 mM) by preparing mock samples containing base LAMP reaction mix components (20 mM Tris-HCl pH=7.9, 20 mM KCI, 60 mM GuCl, 9 mM MgSO₄, 0.1% Tween-20, 0.05% Triton X-100), 25 or 50 µM Zn²⁺/5-Br-PAPS detection reagent and sodium pyrophosphate (concentration range of 0-5 mM).

The PPi concentration-dependent effect on the color of the Zn²⁺/5-Br-PAPS complex was analyzed spectrophotometrically on a Tecan Infinite M1000 Pro Multi-mode Reader (Tecan, Switzerland) by performing a spectral analysis in the 330-600 nm wavelength range while visual color changes were recorded by a cell phone camera.

Figure 3B shows that increasing PPi concentrations progressively change the reaction mix (with 25 µM Zn²⁺/5-Br-PAPS detection reagent) blank-adjusted absorption spectrum. Specifically, increasing PPi concentrations weaken the 560 nm absorption peak with a new 448 nm peak growing simultaneously. Figure 3C demonstrates that this corresponds to an easily discernible visible color change (from magenta to orange-yellow) both with 25 and 50 µM Zn²⁺/5-Br-PAPS detection reagent.

### Example 3: LAMP amplification detection using the Zn²⁺/5-Br-PAPS detection reagent

LAMP amplification reactions (all 20 µl final volume) were performed in a reaction buffer containing 20 mM Tris-HCl pH=7.9, 20 mM KCI, 60 mM GuCl (guanidine hydrochloride), 1.4 mM dNTPs, 0.1% Tween-20, 0.05% Triton X-100 and 320 U/ml *Bst* 2.0 WarmStart^{®} DNA polymerase (M0538, New England Biolabs, Massachusetts, USA). RT-LAMP reactions, where the template strand is RNA, also included 300 U/ml WarmStart^{®} RTx reverse transcriptase (M0380, New England Biolabs, Massachusetts, USA) and 0.8 M betaine. LAMP primer concentrations were the same for all tested primer sets, specifically 1600 nM for FIP/BIP, 200 nM for F3/B3 and 600 nM for LF/LB.

In Figures 4A-B LAMP amplification was performed with primers against human RPP30 and template input of 0.375 ng/µl of human gDNA (G3041, Promega, Wisconsin, USA). An RT-LAMP reaction was also performed with primers against SARS-CoV-2 N gene and template input of 25 copies/µl of synthetic SARS-CoV-2 RNA (102019, Twist Bioscience, California, USA). In both cases reactions were run at 65 °C for 30 minutes. The color transition (from magenta to orange-yellow), which is easily observed by the naked eye, was specific to nucleic acid amplification as only samples with polymerase (e.g., *Bst* 2.0 WarmStart^{®} DNA polymerase and/or WarmStart^{®} RTx reverse transcriptase) changed color (Fig. 4A), while those devoid of polymerase failed to do so (Fig. 4B). This implies that the amount of pyrophosphate produced during LAMP is surprisingly sufficient to displace Zn²⁺ from 5-Br-PAPS. In addition, the type of template (double-stranded DNA/single-stranded RNA) employed had no effect on the success of amplification/color change. Note that the reaction mix is strongly buffered (20 mM Tris-HCl, pH=7.9), which implies that pH change does not contribute to the observed color change.

In Figure 5, a LAMP reaction targeting human RPP30 was performed with template input of 0.375 ng/µl of human gDNA and 50 µM Zn²⁺/5-Br-PAPS detection reagent at 65 °C. Unlike in experiments depicted in Figures 4A-B, all reactions contained *Bst* 2.0 WarmStart^{®} DNA polymerase and, instead of no-polymerase samples, true no-template control (NTC) reactions were run in parallel. Color change was monitored at predetermined intervals during a 90-minute reaction duration. Color started to change at the 15-minute mark and over the span of the next 10 minutes it progressively changed from red to orange-yellow demonstrating that the reagent changes color continuously as nucleic acid amplification is taking place. At the end of the 90-minute period, positive reactions with template were still orange-yellow, while conversely NTC reactions stayed magenta demonstrating that the chelatometric dye 5-Br-PAPS and its Zn²⁺ complex are stable for a prolonged period of time under typical LAMP reaction conditions.

Figures 6A-B and Figure 7 demonstrate that the inclusion of Zn²⁺/5-Br-PAPS detection in the reaction mix does not hamper amplification speed or sensitivity. RT-LAMP targeting SARS-CoV-2 RdRP gene was performed in a standard reaction mix as described earlier supplemented with 0.5 µM of the nucleic acid-intercalating fluorescent dye, SYTO 59 (Thermo Fisher Scientific, Massachusetts, USA) with or without 50 µM Zn²⁺/5-Br-PAPS detection reagent. Different amounts of input template were tested (100, 50, 25, 12.5 and 0 copies/µl of SARS-CoV-2 RNA) and target amplification was monitored in real-time (Cy5 channel) on an AriaMx Real-Time PCR system (Agilent, California, USA). Reactions were run at 65 °C and monitored in real-time for 45 minutes. Figures 6A-B show that reactions with 50 µM of the Zn²⁺/5-Br-PAPS reagent (Fig. 6A) show amplification profiles similar to reactions without it (Fig. 6B, shaded areas are ± SEM) at all template concentrations tested. While time-to-reaction (TTR) mean ± standard deviation (SD) is modestly higher, all samples with a template changed color to orange-yellow irrespective of the input template amount. This demonstrates that colorimetric detection with the Zn²⁺/5-Br-PAPS detection reagent exhibits sensitivity comparable with real-time fluorescent detection.

The following primers were used and sequences between hypens are linkers not complementary to the template nucleic acid:
Human RPP30-F3: GTTGCTCAGGCTGGAG (SEQ ID NO: 1)
Human RPP30-B3: ATCCTAGGAGTTTGAGACCA (SEQ ID NO: 2) 
Human RPP30-FIP: CCGCTGCTTGAGAGGCTGA-CTTTTATT-CAGCTCACTGCAACCT (SEQ
ID NO: 3)
Human RPP30-BIP: TGCACTACCACACCTGGCTATT-AAACATTT-CAACATAGTGAGACCATG (SEQ ID NO: 4)
Human RPP30-LF: AGCGGGAGGATCACTTG (SEQ ID NO: 5)
Human RPP30-LB: TTTTTTCCCTTGTAGAGA (SEQ ID NO: 6)
SARS-CoV-2 N-F3: AACACAAGCTTTCGGCAG (SEQ ID NO: 7)
SARS-CoV-2 N-B3: GAAATTTGGATCTTTGTCATCC (SEQ ID NO: 8) 
SARS-CoV-2 N-FIP: TGCGGCCAATGTTTGTAATCAG-CCAAGGAAATTTTGGGGAC (SEQ ID
NO: 9)
 
SARS-CoV-2 N-BIP: CGCATTGGCATGGAAGTCAC-TTT-GATGGCACCTGTGTAG (SEQ ID
NO: 10)
SARS-CoV-2 N-LF: TTCCTTGTCTGATTAGTTC (SEQ ID NO: 11)
SARS-CoV-2 N-LB: TTCGGGAACGTGGTTGAC (SEQ ID NO: 12)
SARS-CoV-2 RdRP-F3: CTGTGATGCCATGCGAA (SEQ ID NO: 13)
SARS-CoV-2 RdRP-B3: CGGTCAAAGAGTTTTAACCTC (SEQ ID NO: 14) 
SARS-CoV-2 RdRP-FIP: CGTGGTTTGTATGAAATCACCGAAATC-TGTT-
GTATTGTTGGTGTACTGAC (SEQ ID NO: 15)
 
SARS-CoV-2 RdRP-BIP: GACCAGGGCTTTAACTGCAGAG-CTTT-
ATCCCACTTAATGTAAGGC (SEQ ID NO: 16)
SARS-CoV-2 RdRP-LF: AGTTACCATTGAGATCTTGATTATC (SEQ ID NO: 17)
SARS-CoV-2 RdRP-LB: CACATGTTGACACTGACTTAACA (SEQ ID NO: 18)

### Example 4: PCR amplification detection using the Zn²⁺/5-Br-PAPS detection reagent

Reactions containing 25 µM of Zn²⁺/5-Br-PAPS detection reagent were used to detect nucleic acid amplification during PCR on a SensoQuest Gradient Labcycler (SensoQuest, Germany). PCR was performed in a reaction buffer containing 70 mM Tris-HCl pH=7.9, 17.5 mM (NH₄)₂SO₄, 1 mM dNTPs, 3 mM MgCl₂, 0.02% Tween-20 and 50 U/ml HOT FIREpol DNA polymerase (01-02-1000, Solis Biodyne, Estonia). A template input of 0.375 ng/µl of human gDNA and primers targeting human RPP30 (both forward and reverse primers at 500 nM) were used to perform the amplification. The PCR reaction was performed as follows: a 10-minute hot-start at 95 °C was followed by 45 cycles of melting (95 °C for 15 seconds) and annealing/extension (60 °C for 30 seconds). As can be seen in Figure 8, unlike NTC reactions which stayed magenta, reactions with gDNA template changed color from magenta to orange-yellow demonstrating that Zn²⁺/5-Br-PAPS detection reagent can also be used to detect nucleic acid amplification under non-isothermal conditions such as during PCR. Color preservation in both NTC and templated reactions also demonstrates thermal stability of the reagent to repeated cycles of heating up to 95 °C.

The following primers were used:
Human RPP30-forward: AGATTTGGACCTGCGAGCG (SEQ ID NO: 19)
Human RPP30-reverse: GAGCGGCTGTCTCCACAAGT (SEQ ID NO: 20)

### Example 5: Tolerance of the Zn²⁺/5-Br-PAPS detection reagent to reaction buffering and its use in LAMP amplification of buffered samples

It is known from the prior art that colorimetric detection systems for nucleic acid amplification detection, especially those relying on pH-based detection, are susceptible to reaction failure with buffered samples or those containing variable initial pH (e.g., biological samples like saliva), prohibiting their use with unpurified samples. This limitation also precludes proper reaction mix buffering throughout amplification which can result in suboptimal amplification kinetics and/or sensitivity. As the Zn²⁺/5-Br-PAPS detection reagent does not rely on free hydronium ion accumulation with its associated pH decrease to function, reactions utilizing this reagent can not only tolerate strong buffering in the reaction mix but by extension also of the input sample. Such combination of properties lends itself especially well to techniques such as direct pathogen detection with no prior nucleic acid isolation in biological matrices with varying levels of pH/buffering capacity. The following reactions were set up to show that the colorimetric assay of the present invention is robust with respect to pH change, since it accepts buffers preferably higher than 1.5 mM Tris or an equivalent thereto both in the reaction mix and in input samples.

LAMP reactions were performed in the same manner as it is described in Example 3 with some minor modifications in certain experiments which are pointed out in their descriptions. Mock LAMP reactions had the same basic composition as in regular LAMP to which specified amounts of the Zn²⁺/5-Br-PAPS detection reagent was added. To prepare the mock samples, 50% volume of 2x master mix (40 mM Tris-HCl pH=7.9, 40 mM KCI, 120 mM, GuCl, 18 mM MgSO₄, 0.2% Tween-20, 0.1% Triton X-100) was mixed with 25% volume of water or different pH 10 mM buffers (pH = 5, 6 - 10 mM sodium citrate; pH = 7, 8, 9 - 10 mM Tris-HCl; 2.5 mM final concentration) and 25% volume of either water (mock negative sample) or 16 mM sodium pyrophosphate (mock positive sample, 4 mM final concentration).

Figures 9A-B and 10 show the proposed advantages of Zn²⁺/5-Br-PAPS detection reagent versus relying on pH change. LAMP was used to amplify human RPP30 with 0.375 ng/µl of human gDNA as the input and 0.5 µM of SYTO 59 at 65 °C for 30 minutes. An AriaMx Real-Time PCR system was used to monitor the reaction in real-time (Cy5 channel). 50 µM of the Zn²⁺/5-Br-PAPS detection reagent was compared with 50 µM of 5-Br-PAPS only (no Zn²⁺) and 100 µM of Phenol Red (pH-sensitive indicator) with different concentrations of Tris-HCl buffer in the reaction mix. As Figures 9A-B demonstrate, under all conditions tested amplification (as assessed by real-time fluorescent detection) took place if template was present. In addition, higher concentrations of Tris-HCl buffer resulted in faster amplification times (lower TTR) for both Zn²⁺/5-Br-PAPS-based and Phenol Red-based detection (Fig. 9B), and therefore keeping the reaction strongly buffered was found to be advantageous. However, with strong buffering (20 mM Tris-HCl, pH=7.9), pH-dependent detection failed to change color (Fig. 10) despite successful amplification according to real-time detection (Fig. 9A). Contrarily, the Zn²⁺/5-Br-PAPS detection reagent displayed consistent color transition irrespective of Tris-HCl concentration. Note that the observed color change of Zn²⁺/5-Br-PAPS detection reagent is entirely dependent on the presence of Zn²⁺ in the starting reaction mix as 5-Br-PAPS by itself is orange-yellow and does not change color with amplification. In total, the Zn²⁺/5-Br-PAPS detection reagent is not reliant on pH change to detect nucleic acid amplification and works both in very low and high strength buffers.

In Figure 11A, spectral analysis (performed as described in Example 1) of the Zn²⁺/5-Br-PAPS detection reagent (25 µM) can be seen in either mock positive samples (solid lines, with 4 mM PPi) or mock negative samples (dotted lines, no PPi). Replacing 25% of final LAMP reaction mix volume with 10 mM buffer solutions at commonly used pH levels failed to appreciably influence the absorption spectrum in the presence or absence of PPi. As can be seen in Figure 9B, this lack of color change was observable visually as well at both lower and higher concentrations of the Zn²⁺/5-Br-PAPS detection reagent.

In Figures 12A-C LAMP targeting human RPP30 was performed with template input of 0.375 ng/µl of human gDNA, 0.5 µM of SYTO 59 and 50 µM Zn²⁺/5-Br-PAPS detection reagent at 65 °C for 30 minutes. Input gDNA (25% of final reaction volume) was either diluted in water or 10 mM buffers as used for mock sample preparation and target amplification was monitored in real-time (Cy5 channel) on an AriaMx Real-Time PCR system. All replicates with gDNA template amplified successfully irrespective of the diluent (Fig. 12A). While there were some minor differences in time-to-reaction (TTR, Fig. 12B) between individual dissolution media, the inclusion of buffers did not prevent amplification and color change (Fig. 12C) from taking place confirming the robustness of the Zn²⁺/5-Br-PAPS detection reagent with respect to input sample buffering capacity and pH. Indeed, in terms of TTR and reproducibility (i.e., variability between replicates) all tested buffers matched or exceeded the performance of water-only control.

In sum, the inventors could surprisingly show that 5-Br-PAPS in combination with Zinc ions can be used to detect nucleic acid amplification in a reaction mixture.

### Example 6: Use of LNA-comprising primers in LAMP improves time-to-reaction and specificity

The replacement of specific bases with their LNA variants in F3/B3 LAMP primers described herein may significantly alter LAMP reaction kinetics and target recognition. Specifically, introduction of a predefined number of LNA bases near the 5' end of F3/B3 primers significantly reduces time-to-reaction (TTR) while simultaneously increasing specificity, both traits desirable in potential isothermal diagnostic tests. This may be useful to shorten positive reaction times thus widening the window between true and false positive results, which can be problematic for isothermal methods like LAMP. Incorporating LNA-modified bases in LAMP primers is also beneficial for normalizing primer melting temperatures (Tm) and providing flexibility in LAMP primer design when targeting challenging templates that are either A,T or G,C rich or organisms that have high rates of mutations (e.g., SARS-CoV-2 and Influenza). While those skilled in the art may appreciate the fact that LNA incorporation into primers can lead to improved target hybridization rates chiefly by increasing the primer's melting temperature (Tm), due to the complex nature of primer interactions during LAMP target amplification, unthoughtful changes to primer base composition would not be expected to lead to beneficial effects during LAMP amplification and may facilitate non-specific interactions and amplification and impede strand displacement activity of the polymerase.

Unless specified otherwise, in all experiments described below, LAMP amplification was performed as follows. Reaction mixes were prepared by mixing WarmStart Colorimetric RT-LAMP 2X MasterMix (M1800, New England Biolabs, Massachusetts, USA) and 10x primer mix consisting of 6 specific LAMP primers. Base LAMP primer sets targeting SARS-CoV-2 genes E (sets E1, E2, E3), RdRP (set R1) and N (set N1) were designed to follow the recommendations as known from the prior art. Final reaction concentrations of LAMP primers were: FIP/BIP - 1600 nM, F3/B3 - 200 nM, LF/LB - 600 nM. All assays utilized 20 µl reaction volumes. All reactions were also supplemented with 0.05% Triton X-100, 1 mM extra of MgCl₂ (final concentration 9 mM), and 1 µM of the nucleic acid-intercalating dye, SYTO 9 (Thermo Fischer Scientific, Massachusetts, USA). Reactions performed at 65 °C also included 40 mM guanidine isothiocyanate and 0.8 M betaine. Positive test reactions either contained SARS-CoV-2 RNA mixed with human genomic DNA (COV019, Exact Diagnostics, California, USA; when RNA copy/rxn was < 200) or pure SARS-CoV-2 RNA (102019, Twist Bioscience, California, USA; when RNA copy/rxn was ≥ 200). Once the reactions were set up, they were run at a primer set-/condition-specific temperature and allowed to amplify on an Agilent AriaMx Real-Time PCR System (Agilent, California, USA). The process of amplification was continuously monitored every 30 seconds by measuring changes in SYTO 9 fluorescence (FAM channel) and the reaction was terminated after 90 minutes. Time-to-reaction (TTR, cycle threshold / 2) was determined automatically by the Agilent Aria 1.7.1 software (Agilent, California, USA) using default settings. Melt curve analysis of the resulting amplicons was also used to distinguish specific from non-specific products. Specifically, products were considered specific if their Tm was in the range mean ± 0.5 °C of products observed in high template concentration reactions as internally established for each tested primer set. Replicates with no amplification are not depicted in any of the figures and were omitted from calculating descriptive statistics, e.g., mean ± SD.

In Figure 13, four different LAMP primer sets (E1, E2, R1, N1) were used in RT-LAMP to amplify target sequences from the SARS-CoV-2 genome. LNA base substitution at the 5' end of outer primers, F3/B3, was compared with standard LAMP primer sets. Reactions with template included 400 (E1, E2), 100 (R1) or 25 (N1) copies of template per reaction. Reaction temperature was either 60 °C (E1, E2) or 65 °C (R1, N1) and amplification was left to proceed for 90 minutes. Closed symbols represent samples with a template while open symbols represent NTC reactions. Numbers in parenthesis show the number of replicates with templates that successfully amplified. The figure clearly shows that in all tested primer sets the introduction of LNAs near the 5' end improved all investigated metrics of LAMP set performance. Sets with LNAs demonstrated reduced TTR (depicted as fold-change vs. unmodified set) and variability (SD represented by the error bars) as well as unchanged or increased amplification success suggestive of improved sensitivity. Crucially, the propensity towards non-specific amplification was not significantly increased in sets harboring LNA substitutions.

Beneficial effects of 5' LNA incorporation into outer LAMP primers are robust and not dependent on specific bases modified as Figure 14 demonstrates. RT-LAMP was used in conjunction with the set E3 to amplify SARS-CoV-2 E gene (400 copies of template per reaction for the modified sets, 800 copies for the base E3 set) at 60 °C for 90 minutes. Four different combinations of LNA-modified F3/B3 primers (2 F3 primers and 2 B3 primers, labeled [1] or [2]), which differ only in the number/position of LNA bases within their sequence, were compared with the base set with no LNA modifications. As can be seen, every single variant demonstrated improved performance characteristics as described above even though the unmodified set was run with twice as much template.

In Figure 15, RT-LAMP was performed with the set E1 comparing the base set with sets where FIP, BIP, LF and LB were modified with LNAs near their 5' end. In the case of FIP/BIP primers, both F2/B2 and F1c/B1c segments were modified. Reactions included 400 copies of template and were run at 60 °C for 90 minutes. As can be seen, LNA modifications in these primer sets lead to either no change or a significant deterioration of LAMP amplification performance. TTR is higher (slower), variability is higher, and sensitivity (number of amplified replicates) is lower or unchanged. This demonstrates that LNA substitution effects are LAMP primer-specific and are not a result of a generic effect of LNAs on primer hybridization behavior. Summarily, LNAs in primers other than F3/B3 impaired LAMP amplification and/or target recognition and, therefore unlike F3/B3 primers, modification of other LAMP primers is not expected to consistently lead to desirable effects.

The following primers were used, wherein (+N) depicts the corresponding base in form of an LNA and sequences between hypens are linkers not complementary to the template nucleic acid:
SARS-CoV-2 E1-F3: CGTTTCGGAAGAGACAGGT (SEQ ID NO: 21)
SARS-CoV-2 E1-LNA(F3): CG(+T)T(+T)CGGAAGAGACAGGT (SEQ ID NO: 22)
SARS-CoV-2 E1-B3: GACCAGAAGATCAGGAACT (SEQ ID NO: 23)
SARS-CoV-2 E1-LNA(B3): G(+A)CC(+A)G(+A)AGATCAGGAACT (SEQ ID NO: 24) 
SARS-CoV-2 E1-FIP: CGCAGTAAGGATGGCTAGTGT-TTTTT-
ATAGCGTACTTCTTTTTCTTGCTT (SEQ ID NO: 25)
 
SARS-CoV-2 E1-LNA(FIP): CGC(+A)G(+T)AAGGATGGCTAGTGT-TTTTT-
(+A)(+T)AGCG(+T)ACTTCTTTTTCTTGC (SEQ ID NO: 26)
 
SARS-CoV-2 E1-BIP: CTTCGATTGTGTGCGTACTGCTG-T-TTCAGATTTTTAACACGAGAG
(SEQ ID NO: 27)
 
SARS-CoV-2 E1-LNA(BIP): CT(+T)CGATTGTGTGCGTACTGCTG-T-
(+T)(+TT)CAG(+A)(+T)TTTTAACACGAGAG (SEQ ID NO: 28)
SARS-CoV-2 E1-LF: ACTAGCAAGAATACCACGAA (SEQ ID NO: 29)
SARS-CoV-2 E1-LNA(LF): AC(+T)(+A)GC(+A)AGATAACCACGAA (SEQ ID NO: 30)
SARS-CoV-2 E1-LB: CGTGAGTCTTGTAAAACCTTC (SEQ ID NO: 31)
SARS-CoV-2 E1-LNA(LB): CG(+T)G(+A)GTCTTGTAAAACCTTC (SEQ ID NO: 32)
SARS-CoV-2 E2-F3: TGTACTCATTCGTTTCGGAAG (SEQ ID NO: 33)
SARS-CoV-2 E2-LNA(F3): TG(+T)AC(+T)CATTCGTTTCGGAAG (SEQ ID NO: 34)
SARS-CoV-2 E2-B3: CAGGAACTCTAGAAGAATTCAGA (SEQ ID NO: 35)
SARS-CoV-2 E2-LNA(B3): C(+A)G(+G)A(+A)CTCTAGAAGAATTCAGA (SEQ ID NO: 36) 
SARS-CoV-2 E2-FIP: CGCAGTAAGGATGGCTAGTGTA-TTTT-GCGTACTTCTTTTTCTTGCTT
(SEQ ID NO: 37)
 
SARS-CoV-2 E2-LNA(FIP): CGC(+A)G(+T)AAGGATGGCTAGTGTA-TTTT-
GCG(+T)(+A)CTTCTTTTTCTTGCTT (SEQ ID NO: 38)
 
SARS-CoV-2 E2-BIP: CTTCGATTGTGTGCGTACTGC-ATTT-
CGAGAGTAAACGTAAAAAGAAGGT (SEQ ID NO: 39)
 
SARS-CoV-2 E2-LNA(BIP): C(+T)(+T)CGATTGTGTGCGTACTGC-ATTT-
CG(+A)GAGTAAACGTAAAAAGAAGGT (SEQ ID NO: 40)
SARS-CoV-2 E2-LF: ACTAGCAAGAATACCACGAA (SEQ ID NO: 41)
SARS-CoV-2 E2-LNA(LF): (+A)C(+T)(+A)GCAAGAATACCACGA (SEQ ID NO: 42)
SARS-CoV-2 E2-LB: CGTGAGTCTTGTAAAACCTTC (SEQ ID NO: 43)
SARS-CoV-2 E2-LNA(LB): (+T)GC(+A)A(+T)ATTGTTAACGTGAG (SEQ ID NO: 44)
SARS-CoV-2 E3-F3: TACTCATTCGTTTCGGAAGAG (SEQ ID NO: 45)
SARS-CoV-2 E3-LNA(F3) [1]: (+T)AC(+T)CATTCGTTTCGGAAGAG (SEQ ID NO: 46)
SARS-CoV-2 E3-LNA(F3) [2]: T(+A)CTC(+A)TTCGTTTCGGAAGAG (SEQ ID NO: 47)
SARS-CoV-2 E3-B3: CAGGAACTCTAGAAGAATTCAGA (SEQ ID NO: 48)
SARS-CoV-2 E3-LNA(B3) [1]: C(+A)G(+G)A(+A)CTCTAGAAGAATTCAGA (SEQ ID NO: 49)
SARS-CoV-2 E3-LNA(B3) [2]: C(+A)GG(+A)ACTCTAGAAGAATTCAGA (SEQ ID NO: 50) 
SARS-CoV-2 E3-FIP: GTAAGGATGGCTAGTGTAACTAGC-
CAGGTACGTTAATAGTTAATAGCG (SEQ ID NO: 51)
 
SARS-CoV-2 E3-BIP: GCTTCGATTGTGTGCGTACT-CGAGAGTAAACGTAAAAAGAAGG
(SEQ ID NO: 52)
SARS-CoV-2 E3-LF: CCACGAAAGCAAGAAAAAGAAG (SEQ ID NO: 53)
SARS-CoV-2 E3-LB: GCTGCAATATTGTTAACGTGAG (SEQ ID NO: 54)
SARS-CoV-2 R1-F3: CTGTGATGCCATGCGAA (SEQ ID NO: 55)
SARS-CoV-2 R1-LNA(F3): C(+T)G(+T)GATGCCATGCGAA (SEQ ID NO: 56)
SARS-CoV-2 R1-B3: CGGTCAAAGAGTTTTAACCTC (SEQ ID NO: 57)
SARS-CoV-2 R1-LNA(B3): C(+G)G(+T)CA(+A)AGAGTTTTAACCTC (SEQ ID NO: 58) 
SARS-CoV-2 R1-FIP: CGTGGTTTGTATGAAATCACCGAAATC-TGTT-
GTATTGTTGGTGTACTGAC (SEQ ID NO: 59)
 
SARS-CoV-2 R1-BIP: GACCAGGGCTTTAACTGCAGAG-CTTT-ATCCCACTTAATGTAAGGC
(SEQ ID NO: 60)
SARS-CoV-2 R1-LF: AGTTACCATTGAGATCTTGATTATC (SEQ ID NO: 61)
SARS-CoV-2 R1-LB: CACATGTTGACACTGACTTAACA (SEQ ID NO: 62)
SARS-CoV-2 N1-F3: AACACAAGCTTTCGGCAG (SEQ ID NO: 63)
SARS-CoV-2 N1-LNA(F3): A(+A)C(+A)CAAGCTTTCGGCAG (SEQ ID NO: 64)
SARS-CoV-2 N1-B3: GAAATTTGGATCTTTGTCATCC (SEQ ID NO: 65)
SARS-CoV-2 N1-LNA(B3): G(+A)(+A)ATT(+T)GGATCTTTGTCATCC (SEQ ID NO: 66) 
SARS-CoV-2 N1-FIP: TGCGGCCAATGTTTGTAATCAG-CCAAGGAAATTTTGGGGAC (SEQ
ID NO: 67)
 
SARS-CoV-2 N1-BIP: CGCATTGGCATGGAAGTCAC-TTT-GATGGCACCTGTGTAG (SEQ ID
NO: 68)
SARS-CoV-2 N1-LF: TTCCTTGTCTGATTAGTTC (SEQ ID NO: 69)
SARS-CoV-2 N1-LB: ACCTTCGGGAACGTGGTT (SEQ ID NO: 70)

## Claims

1. A loop mediated isothermal amplification (LAMP) method, comprising
synthesizing a nucleic acid sequence from a template nucleic acid sequence in a reaction mixture comprising
(i) a first inner primer (FIP) nucleotide sequence and a second inner primer (BIP) nucleotide sequence,
(ii) a first outer primer (F3) nucleotide sequence and a second outer primer (B3) nucleotide sequence, wherein said F3 nucleotide sequence and/or said B3 nucleotide sequence comprises one or more locked nucleic acid (LNA) nucleotides which are located within the first third of said F3 nucleotide sequence or said B3 nucleotide sequence, respectively, wherein said first third is the first third of a nucleotide sequence in 5'- to 3'-orientation,
(iii) a DNA polymerase catalyzing a strand-displacement-type reaction of synthesizing a complementary nucleic acid strand from said template nucleic acid, and
(iv) nucleotides serving as substrates for said DNA polymerase
at such a temperature that at least FIP nucleotide sequence and BIP nucleotide sequence can form stable base pairing with its complementary nucleotide sequences comprised by said template nucleic acid while the DNA polymerase activity can be maintained, thereby synthesizing a nucleic acid sequence.

2. The method of claim 1,
(i) wherein said FIP nucleotide sequence comprises at least two regions F2 and F1c, wherein said F1c region is linked to the 5'-side of the F2 region,
wherein said F2 region has a nucleotide sequence complementary to an arbitrary region F2c in said template nucleic acid sequence, and
wherein said F1c region has substantially the same nucleotide sequence as a region F1c located at the 5'-side of said F2c region in said template nucleic acid sequence;
(ii) wherein said BIP nucleotide sequence comprises at least two regions B2 and B1c,
wherein said B1c region is linked to the 5'-side of the B2 region,
wherein said B2 region has a nucleotide sequence complementary to an arbitrary region B2c in said template nucleic acid sequence strand, and
wherein said B1c region has substantially the same nucleotide sequence as a region B1c located at the 5'-side of said region B2c in said template nucleic acid sequence;
(iii) wherein said F3 nucleotide sequence has a nucleotide sequence substantially complementary to a region F3c in said template nucleic acid sequence,
wherein region F3c is located at the 3'-side of region F2c in said template nucleic acid sequence,
wherein region F2c is located at the 3'-side of region F1c in said template nucleic acid sequence; and/or
(iv) wherein said B3 nucleotide sequence has a nucleotide sequence substantially complementary to a region B3c in the template nucleic acid sequence,
wherein region B3c is located at the 3'-side of region B2c in said template nucleic acid sequence
wherein region B2c is located at the 3'-side of region B1c in said template nucleic acid sequence.

3. The method of any one of the preceding claims,
(i) wherein said reaction mixture further comprises loop primer nucleotide sequence F and/or loop primer nucleotide sequence B;
(ii) wherein said reaction mixture further comprises stem primer nucleotide sequence F and/or stem primer nucleotide sequence B;
(iii) wherein said reaction mixture further comprises swarm primer nucleotide sequence F1S and/or swarm primer nucleotide sequence B1S;
(iv) wherein said reaction mixture further comprises at least a further first inner primer (FIP2) nucleotide sequence and a further second inner primer (BIP2) nucleotide sequence, both of which are different from said FIP and BIP nucleotide sequence;
(v) wherein said reaction mixture further comprises primer nucleotide sequences for performing said method as multiple cross displacement amplification LAMP; and/or
(vi) wherein said reaction mixture further comprises primer nucleotide sequences for performing said method as reverse transcription isothermal multiple self matching LAMP.

4. The method of any one of the preceding claims,
(i) wherein said template nucleic acid sequence is single stranded or double stranded;
(ii) wherein said template nucleic acid sequence is single- or double-stranded DNA, RNA or a DNA/RNA chimera;
(iii) wherein said DNA polymerase is a DNA-dependent DNA polymerase or an RNA-dependent DNA polymerase;
(iv) wherein said DNA polymerase has reverse transcriptase activity.

5. The method of any one of the preceding claims,
(i) wherein said F3 nucleotide sequence has 15-30, preferably 17-25 nucleotides in length; and/or
(ii) wherein said B3 nucleotide sequence has 15-30, preferably 17-25 nucleotides in length.

6. The method of any one of the preceding claims,
(i) wherein said F3 nucleotide sequence comprises 1-5, preferably 3 LNA nucleotides; and/or
(ii) wherein said B3 nucleotide sequence comprises 1-5, preferably 3 LNA nucleotides.

7. The method of any one of the preceding claims, wherein the reaction mixture further comprises a regulator for melting temperature, e.g., betaine, preferably in a concentration between 0.2 M to 3.0 M, Tween-20/Triton-X, preferably in a concentration between 0.02 % to 0.2 %, guanidine thiocyanate or hydrochloride, preferably in a concentration between 20 mM and 80 mM, single-stranded binding protein (SSB), BSA, preferably in a concentration between 0.02 mg/ml to 2 mg/ml, TMAC, preferably in a concentration between 5 mM and 80 mM, and/or TCEP/DTT, preferably in a concentration between 0.5 mM to 5 mM.

8. The method of any one of the preceding claims, wherein said reaction mixture further comprises a detector for detecting the product of the nucleic acid sequence synthesis reaction, preferably wherein said detector is a metallochromic indicator, more preferably wherein the detector further comprises a transition metal or post-transition metal.

9. The method of any one of the preceding claims, wherein the detector is a metallochromic indicator, preferably 2-(5-Bromo-2-pyridylazo)-5-[N-propyl-N-(3-sulfopropyl)amino]phenol (5-Br-PAPS), PAR or Zincon and in combination with metal ions, preferably transition metal or post-transition metal ions, more preferably Zn²⁺, Cu²⁺, Co²⁺, Ni²⁺, Pt²⁺, Ru²⁺, Rh²⁺, and/or Fe²⁺ ions, most preferably Zn²⁺ ions, said metallochromic indicator and each of said metal ions building a complex, but not building a complex with magnesium, and
wherein the method further comprises
(b) detecting a change in spectral or fluorescent properties of the complex resulting from the amplification of said nucleic acid sequence.

10. The method of any one of the preceding claims, wherein said method is for
(a) synthesizing a nucleic acid sequence,
(b) detecting a nucleic acid sequence, or
(c) diagnosing a disease, preferably caused by a pathogen.

11. An *in vitro* method for detecting a nucleic acid sequence amplification product, comprising
(a) contacting a reaction mixture comprising a template nucleic acid sequence, primer nucleotide sequences, nucleotides and a polymerase capable of amplifying said nucleic acid molecule with a metallochromic indicator and a transition or post-transition metal ion, said metallochromic indicator and said transition or post-transition metal ion building a complex, but not building a complex with magnesium;
(b) amplifying said nucleic acid sequence under suitable conditions to obtain a nucleic acid sequence amplification product;
(c) detecting a change in spectral properties of the complex resulting from the amplification of said nucleic acid sequence, wherein formation of said complex comprising said metallochromic indicator and said transition or post-transition metal ion leads to a color change of the solution;
wherein the amplification is performed by the method as defined in any one of claims 1-7.

12. The method of claim 11,
(i) wherein said metallochromic indicator is 2-(5-Bromo-2-pyridylazo)-5-[N-propyl-N-(3-sulfopropyl)amino]phenol (5-Br-PAPS);
(ii) wherein said metal ion is a Zn²⁺ ion; and/or
(iii) wherein said change in spectral properties of said complex is in the spectrum from 380 to 740 nm wavelength.

13. A kit for the synthesis of a nucleic acid sequence by loop mediated isothermal amplification (LAMP) on a template nucleic acid sequence, comprising
(i) a first outer primer (F3) nucleotide sequence and a second outer primer (B3) nucleotide sequence, each of which comprises locked nucleic acid (LNA) nucleotides, which are located within the first third of said F3 nucleotide sequence or said B3 nucleotide sequence, respectively, wherein said first third is the first third of a nucleotide sequence in 5'- to 3'-orientation
(ii) a first inner primer (FIP) nucleotide sequence and a second inner primer (BIP) nucleotide sequence,
(iii) a DNA polymerase catalyzing a strand-displacement-type reaction of synthesizing a complementary nucleic acid strand from said template nucleic acid,
(iv) nucleotides serving as substrates for said DNA polymerase, and optionally
(v) a metallochromic indicator, preferably 5-Br-PAPS, PAR or Zincon; and/or optionally
(vi) metal ions, preferably Zn²⁺ ions.

14. Use of the kit of claim 13 for performing the method of any one of claims 1 to 12.

## Patentansprüche

1. Verfahren zur Schleifen-vermittelten isothermen Amplifikation (LAMP), umfassend:
Synthese einer Nukleinsäuresequenz aus einer Matrizen-Nukleinsäuresequenz in einer Reaktionsmischung, umfassend:
(i) einer ersten inneren Primer (FIP)-Nukleotidsequenz und einer zweiten inneren Primer (BIP)-Nukleotidsequenz,
(ii) einer ersten äußeren Primer (F3)-Nukleotidsequenz und einer zweiten äußeren Primer (B3)-Nukleotidsequenz, wobei die besagte F3-Nukleotidsequenz und/oder die besagte B3-Nukleotidsequenz ein oder mehrere verbrückte Nukleinsäure (LNA)-Nukleotide umfasst, die sich im ersten Drittel der F3-Nukleotidsequenz oder der B3-Nukleotidsequenz befinden, wobei das besagte erste Drittel das erste Drittel einer Nukleotidsequenz in 5'-3'-Richtung ist,
(iii) einer DNS-Polymerase, die eine strangverdrängende Reaktion katalysiert, bei der ein komplementärer Nukleinsäurestrang von der Matrizen-Nukleinsäure synthetisiert wird, und
(iv) Nukleotide, die als Substrate für die DNS-Polymerase dienen
bei einer Temperatur, bei der zumindest die FIP-Nukleotidsequenz und die BIP-Nukleotidsequenz eine stabile Basenpaarung mit ihren komplementären Nukleotidsequenzen, die die besagte Matrizen-Nukleinsäure umfasst, bilden können, während die Aktivität der DNS-Polymerase aufrechterhalten wird, wodurch eine Nukleinsäuresequenz synthetisiert wird.

2. Verfahren nach Anspruch 1,
(i) wobei die FIP-Nukleotidsequenz mindestens zwei Regionen F2 und F1c umfasst, wobei die F1c-Region an der 5'-Seite der F2-Region gebunden ist,
wobei die F2-Region eine Nukleotidsequenz aufweist, die komplementär zu einer beliebigen F2c-Region in der besagten Matrizen-Nukleinsäuresequenz ist, und
wobei die F1c-Region im Wesentlichen dieselbe Nukleotidsequenz wie eine F1c-Region aufweist, die sich an der 5'-Seite der F2c-Region in der besagten Matrizen-Nukleinsäuresequenz befindet;
(ii) wobei die BIP-Nukleotidsequenz mindestens zwei Regionen B2 und B1c umfasst,
wobei die B1c-Region an der 5'-Seite der B2-Region gebunden ist,
wobei die B2-Region eine Nukleotidsequenz aufweist, die zu einer beliebigen Region B2c in dem Matrizenstrang der besagten Nukleinsäure komplementär ist, und
wobei die B1c-Region im Wesentlichen dieselbe Nukleotidsequenz wie eine B1c-Region aufweist, die sich an der 5'-Seite der B2c-Region in der besagten Matrizen-Nukleinsäuresequenz befindet;
(iii) wobei die F3-Nukleotidsequenz eine Nukleotidsequenz aufweist, die im Wesentlichen komplementär zu einer F3c-Region in der besagten Matrizen-Nukleinsäuresequenz ist,
wobei die F3c-Region sich an der 3'-Seite der F2c-Region in der besagten Matrizen-Nukleinsäuresequenz befindet,
wobei die F2c-Region sich an der 3'-Seite der F1c-Region in der besagten Matrizen-Nukleinsäuresequenz befindet; und/oder
(iv) wobei die B3-Nukleotidsequenz eine Nukleotidsequenz aufweist, die im Wesentlichen komplementär zu einer B3c-Region in der Matrizen-Nukleinsäuresequenz ist,
wobei die B3c-Region sich an der 3'-Seite der B2c-Region in der besagten Matrizen-Nukleinsäuresequenz befindet,
wobei die B2c-Region sich an der 3'-Seite der B1c-Region in der besagten Matrizen-Nukleinsäuresequenz befindet.

3. Verfahren nach einem der vorhergehenden Ansprüche,
(i) wobei die Reaktionsmischung weiterhin einer Schleifen-Primer-Nukleotidsequenz F und/oder einer Schleifen-Primer-Nukleotidsequenz B umfasst;
(ii) wobei die Reaktionsmischung weiterhin einer Stamm-Primer-Nukleotidsequenz F und/oder einer Stamm-Primer-Nukleotidsequenz B umfasst;
(iii) wobei die Reaktionsmischung weiterhin einer Schwarm-Primer-Nukleotidsequenz F1S und/oder einer Schwarm-Primer-Nukleotidsequenz B1S umfasst;
(iv) wobei die Reaktionsmischung weiterhin mindestens einer weiteren ersten inneren Primer-Nukleotidsequenz (FIP2) und einer weiteren zweiten inneren Primer-Nukleotidsequenz (BIP2) umfasst, die sich beide von der FIP- und BIP-Nukleotidsequenz unterscheiden;
(v) wobei die Reaktionsmischung weiterhin Primer-Nukleotidsequenzen zur Durchführung des Verfahrens als Mehrfache Kreuzverdrängungsamplifikation LAMP umfasst; und/oder
(vi) wobei die Reaktionsmischung ferner Primer-Nukleotidsequenzen zur Durchführung des Verfahrens als isotherme reverse Transkription mit mehrfacher Selbstanpassung (LAMP) umfasst.

4. Verfahren nach einem der vorhergehenden Ansprüche,
(i) wobei die besagte Matrizen-Nukleinsäuresequenz einzelsträngig oder doppelsträngig ist;
(ii) wobei die besagte Matrizen-Nukleinsäuresequenz einzel- oder doppelsträngige DNS, RNS oder ein DNS/RNS-Chimär ist;
(iii) wobei die DNS-Polymerase eine DNA-abhängige DNS-Polymerase oder eine RNAabhängige DNS-Polymerase ist;
(iv) wobei die DNS-Polymerase eine Reverse Transkriptase-Aktivität aufweist.

5. Verfahren nach einem der vorhergehenden Ansprüche,
(i) wobei die F3-Nukleotidsequenz eine Länge von 15-30, vorzugsweise 17-25, Nukleotiden aufweist; und/oder
(ii) wobei die B3-Nukleotidsequenz eine Länge von 15-30, vorzugsweise 17-25, Nukleotiden aufweist.

6. Verfahren nach einem der vorhergehenden Ansprüche,
(i) wobei die F3-Nukleotidsequenz 1-5, vorzugsweise 3, verbrückte Nukleinsäure (LNA)-Nukleotide umfasst; und/oder
(ii) wobei die B3-Nukleotidsequenz 1-5, vorzugsweise 3, verbrückte Nukleinsäure (LNA)-Nukleotide umfasst.

7. Verfahren nach einem der vorhergehenden Ansprüche, wobei die Reaktionsmischung weiterhin einen Regulator der Schmelztemperatur umfasst, zum Beispiel, Betain, vorzugsweise in einer Konzentration zwischen 0,2 M bis 3,0 M, Tween-20/Triton-X, vorzugsweise in einer Konzentration zwischen 0,02 % bis 0,2 %, Guanidinthiocyanat oder -hydrochlorid, vorzugsweise in einer Konzentration zwischen 20 mM und 80 mM, Einzelstrang-bindendes Protein (SSB), BSA, vorzugsweise in einer Konzentration zwischen 0,02 mg/ml bis 2 mg/ml, TMAC, vorzugsweise in einer Konzentration zwischen 5 mM und 80 mM, und/oder TCEP/DTT, vorzugsweise in einer Konzentration zwischen 0,5 mM bis 5 mM.

8. Verfahren nach einem der vorhergehenden Ansprüche, wobei die Reaktionsmischung weiterhin einen Detektor zum Nachweis des Produkts der Nukleinsäuresequenz-Synthesereaktion umfasst, vorzugsweise wobei der Detektor ein metallochromischer Indikator ist, und noch vorzugsweiser wobei der Detektor weiterhin ein Übergangsmetall oder ein Post-Übergangsmetall umfasst.

9. Verfahren nach einem der vorhergehenden Ansprüche, wobei der Detektor ein metallochromer Indikator ist, vorzugsweise 2-(5-Brom-2-pyridylazo)-5-[N-Propyl-N-(3-sulfopropyl)amino]phenol (5-Br-PAPS), PAR oder Zincon, und in Kombination mit Metallionen, vorzugsweise Übergangsmetall- oder Post-Übergangsmetall-Ionen, besonders bevorzugt Zn²⁺, Cu²⁺, Co²⁺, Ni²⁺, Pt²⁺, Ru²⁺, Rh²⁺ und/oder Fe²⁺-Ionen, am meisten bevorzugt Zn²⁺-Ionen, wobei der metallochrome Indikator und jedes der Metallionen einen Komplex bilden, jedoch keinen Komplex mit Magnesium bilden, und wobei das Verfahren ferner umfasst
(b) das Detektieren einer Änderung der spektralen oder fluoreszierenden Eigenschaften des Komplexes, die sich aus der Amplifikation der besagten Nukleinsäure ergibt.

10. Verfahren nach einem der vorhergehenden Ansprüche, wobei das Verfahren dient zur
(a) Synthese einer Nukleinsäuresequenz,
(b) Nachweis einer Nukleinsäuresequenz, oder
(c) Diagnose einer Krankheit, vorzugsweise verursacht durch einen Pathogen.

11. In vitro Verfahren zum Nachweis eines Nukleinsäuresequenz-Amplifikationsprodukts, umfassend:
(a) das Inkontaktbringen einer Reaktionsmischung umfassend: einer Matrizen-Nukleinsäuresequenz, Primer-Nukleotidsequenzen, Nukleotide und einer Polymerase, die in der Lage ist, die besagte Nukleinsäuremolekül mit einem metallochromen Indikator und einem Übergangs- oder Post-Übergangsmetallion zu amplifizieren, wobei der besagte metallochrome Indikator und das besagte Übergangs- oder Post-Übergangsmetallion einen Komplex bilden, jedoch keinen Komplex mit Magnesium;
(b) die Amplifikation der besagten Nukleinsäuresequenz unter geeigneten Bedingungen zur Gewinnung eines Nukleinsäuresequenz-Amplifikationsprodukts;
(c) das Nachweisen einer Änderung der spektralen Eigenschaften des Komplexes, die sich aus der Amplifikation der besagten Nukleinsäuresequenz ergibt, wobei die Bildung des besagten Komplexes, umfassend dem metallochromen Indikator und dem Übergangs- oder Post-Übergangsmetallion, zu einer Farbänderung der Lösung führt;
wobei die Amplifikation nach dem Verfahren gemäß einem der Ansprüche 1-7 durchgeführt wird.

12. Verfahren nach Anspruch 11,
(i) wobei der besagte metallochrome Indikator 2-(5-Bromo-2-pyridylazo)-5-[N-propyl-N-(3-sulfopropyl)amino]phenol (5-Br-PAPS) ist;
(ii) wobei das besagte Metallion ein Zn²⁺-Ion ist; und/oder
(iii) wobei die besagte Änderung der spektralen Eigenschaften des Komplexes im Spektrum von 380 bis 740 nm Wellenlänge liegt.

13. Kit zur Synthese einer Nukleinsäuresequenz durch Schleifen-vermittelte isotherme Amplifikation (LAMP) auf einer Matrizen-Nukleinsäuresequenz, umfassend:
(i) einer ersten äußeren Primer-Nukleotidsequenz (F3) und einer zweiten äußerne Primer-Nukleotidsequenz (B3), die jeweils verbrückte Nukleinsäure (LNA)-Nukleotide umfassen, die sich im ersten Drittel der F3-Nukleotidsequenz oder der B3-Nukleotidsequenz befinden, wobei das besagte erste Drittel das erste Drittel einer Nukleotidsequenz in 5'-3'-Richtung ist;
(ii) einer ersten inneren Primer-Nukleotidsequenz (FIP) und einer zweiten inneren Primer-Nukleotidsequenz (BIP),
(iii) einer DNS-Polymerase, die eine strangverdrängende Reaktion katalysiert, bei der ein komplementärer Nukleinsäurestrang von der besagten Matrizen-Nukleinsäure synthetisiert wird,
(iv) Nukleotide, die als Substrate für die DNS-Polymerase dienen; und optional
(v) einen metallochromen Indikator, vorzugsweise 5-Br-PAPS, PAR oder Zincon; und/oder optional
(vi) Metallionen, vorzugsweise Zn²⁺-Ionen.

14. Verwendung des Kits nach Anspruch 13 zur Durchführung des Verfahrens nach einem der Ansprüche 1 bis 12.

## Revendications

1. Méthode d'amplification isotherme à médiation par boucle (LAMP) comprenant
la synthèse d'une séquence d'acide nucléique à partir d'une séquence d'acide nucléique matrice dans un mélange réactionnel comprenant
(i) une première séquence nucléotidique d'amorce interne (FIP) et une deuxième séquence nucléotidique d'amorce interne (BIP),
(ii) une première séquence nucléotidique d'amorce externe (F3) et une deuxième séquence nucléotidique d'amorce externe (B3), dans laquelle ladite séquence nucléotidique F3 et/ou ladite séquence nucléotidique B3 comprend un ou plusieurs nucléotides d'acide nucléique verrouillé (LNA) qui sont situés dans le premier tiers de ladite séquence nucléotidique F3 ou de ladite séquence nucléotidique B3, respectivement, dans laquelle ledit premier tiers est le premier tiers d'une séquence nucléotidique orientée de 5'- à 3'-direction,
(iii) une ADN polymérase catalysant une réaction de type déplacement de brin pour synthétiser un brin d'acide nucléique complémentaire à partir de l'acide nucléique matrice, et
(iv) des nucléotides servant de substrats à ladite ADN polymérase
à une température telle qu'au moins la séquence de nucléotides FIP et la séquence de nucléotides BIP peuvent former un appariement de base stable avec leurs séquences de nucléotides complémentaires comprises dans ledit acide nucléique matrice tout en maintenant l'activité de l'ADN polymérase, synthétisant ainsi une séquence d'acide nucléique.

2. Méthode de la revendication 1,
(i) dans laquelle ladite séquence nucléotidique FIP comprend au moins deux régions F2 et F1c, dans laquelle ladite région F1c est liée au côté 5'- de la région F2,
dans laquelle ladite région F2 possède une séquence nucléotidique complémentaire d'une région arbitraire F2c dans ladite séquence d'acide nucléique matrice, et
dans laquelle ladite région F1c a sensiblement la même séquence nucléotidique qu'une région F1c située du côté 5'de ladite région F2c dans ladite séquence d'acide nucléique matrice;
(ii) dans laquelle ladite séquence nucléotidique BIP comprend au moins deux régions B2 et B1c,
dans laquelle ladite région B1c est liée au côté 5'- de la région B2,
dans laquelle ladite région B2 possède une séquence nucléotidique complémentaire d'une région arbitraire B2c dans ledit brin de séquence d'acide nucléique matrice, et
dans laquelle ladite région B1c a sensiblement la même séquence nucléotidique qu'une région B1c située du côté 5'de ladite région B2c dans ladite séquence d'acide nucléique matrice;
(iii) dans laquelle ladite séquence nucléotidique F3 a une séquence nucléotidique sensiblement complémentaire d'une région F3c dans ladite séquence d'acide nucléique matrice,
dans laquelle la région F3c est située du côté 3'de la région F2c dans ladite séquence d'acide nucléique matrice,
dans laquelle la région F2c est située du côté 3'de la région F1c dans ladite séquence d'acide nucléique matrice; et/ou
(iv) dans laquelle ladite séquence nucléotidique B3 possède une séquence nucléotidique sensiblement complémentaire d'une région B3c dans la séquence d'acide nucléique matrice,
dans laquelle la région B3c est située du côté 3'de la région B2c dans ladite séquence d'acide nucléique matrice
dans laquelle la région B2c est située du côté 3'- de la région B1c dans ladite séquence d'acide nucléique matrice.

3. Méthode de l'une quelconque des revendications précédentes,
(i) dans lequel ledit mélange réactionnel comprend en outre une séquence nucléotidique d'amorce de boucle F et/ou une séquence nucléotidique d'amorce de boucle B;
(ii) dans lequel ledit mélange réactionnel comprend en outre la séquence nucléotidique d'amorce de tige F et/ou la séquence nucléotidique d'amorce de tige B;
(iii) dans lequel ledit mélange réactionnel comprend en outre la séquence nucléotidique d'amorce d'essaim F1S et/ou la séquence nucléotidique d'amorce d'essaim B1S;
(iv) dans lequel ledit mélange réactionnel comprend en outre au moins une autre séquence nucléotidique de la première amorce interne (FIP2) et une autre séquence nucléotidique de la deuxième amorce interne (BIP2), toutes deux différentes des séquences nucléotidiques FIP et BIP;
v) dans lequel ledit mélange réactionnel comprend en outre des séquences nucléotidiques d'amorces pour la mise en oeuvre de ladite méthode en tant qu'amplification par déplacement croisé multiple (LAMP); et/ou
vi) dans lequel ledit mélange réactionnel comprend en outre des séquences nucléotidiques d'amorces pour la mise en oeuvre de ladite méthode en tant qu'amplification par déplacement croisé multiple (LAMP) par transcription inverse isotherme.

4. Méthode de l'une quelconque des revendications précédentes,
(i) dans laquelle ladite séquence d'acide nucléique modèle est simple ou double;
(ii) dans laquelle ladite séquence d'acide nucléique matrice est un ADN simple ou double brin, un ARN ou une chimère ADN/ARN;
(iii) dans laquelle ladite ADN polymérase est une ADN polymérase dépendante de l'ADN ou une ADN polymérase dépendante de l'ARN;
(iv) dans laquelle ladite ADN polymérase a une activité de transcriptase inverse.

5. Méthode de l'une des revendications précédentes,
(i) dans laquelle ladite séquence de nucléotides F3 a une longueur de 15 à 30, de préférence de 17 à 25, nucléotides; et/ou
(ii) dans laquelle ladite séquence de nucléotides B3 a une longueur de 15 à 30 nucléotides, de préférence de 17 à 25, nucléotides.

6. Méthode de l'une quelconque des revendications précédentes,
(i) dans laquelle ladite séquence de nucléotides F3 comprend de 1 à 5, de préférence 3 nucléotides LNA; et/ou
(ii) dans laquelle ladite séquence de nucléotides B3 comprend de 1 à 5, de préférence 3 nucléotides LNA.

7. Méthode de l'une quelconque des revendications précédentes, dans laquelle le mélange réactionnel comprend en outre un régulateur de la température de fusion, par ex, bétaïne, de préférence à une concentration comprise entre 0,2 M et 3,0 M, Tween-20/Triton-X, de préférence à une concentration comprise entre 0,02 % et 0,2 %, thiocyanate ou chlorhydrate de guanidine, de préférence à une concentration comprise entre 20 mM et 80 mM, protéine de liaison simple brin (SSB), BSA, de préférence à une concentration comprise entre 0. 02 mg/ml à 2 mg/ml, TMAC, de préférence à une concentration comprise entre 5 mM et 80 mM, et/ou TCEP/DTT, de préférence à une concentration comprise entre 0,5 mM et 5 mM.

8. Méthode de l'une quelconque des revendications précédentes, dans laquelle ledit mélange réactionnel comprend en outre un détecteur pour détecter le produit de la réaction de synthèse de séquence d'acide nucléique, de préférence dans lequel ledit détecteur est un indicateur métallochromique, plus préférentiellement dans lequel le détecteur comprend en outre un métal de transition ou un métal de post-transition.

9. Méthode de l'une quelconque des revendications précédentes, dans laquelle le détecteur est un indicateur métallochromique, de préférence le 2-(5-Bromo-2-pyridylazo)-5-[N-propyl-N-(3-sulfopropyl)amino]phénol (5-Br-PAPS), le PAR ou le Zincon et en combinaison avec des ions métalliques, de préférence des ions de métaux de transition ou de métaux de post-transition, de préférence des ions Zn²⁺, Cu²⁺, Co²⁺, Ni²⁺, Pt²⁺, Ru²⁺, Rh²⁺ et/ou Fe²⁺, de préférence des ions Zn²⁺, ledit indicateur métallochromique et chacun desdits ions métalliques formant un complexe, mais ne formant pas de complexe avec le magnésium, et
dans laquelle la méthode comprend en outre
(b) la détection d'un changement dans les propriétés spectrales ou fluorescentes du complexe résultant de l'amplification de ladite séquence d'acide nucléique.

10. Méthode de l'une quelconque des revendications précédentes, dans laquelle ladite méthode est destinée à
(a) synthétiser une séquence d'acide nucléique,
(b) détecter une séquence d'acide nucléique, ou
(c) diagnostiquer une maladie, de préférence causée par un agent pathogène.

11. *In vitro* Méthode de détection d'un produit d'amplification d'une séquence d'acide nucléique, comprenant
(a) mettre en contact un mélange réactionnel comprenant une séquence d'acide nucléique matrice, des séquences nucléotidiques d'amorces, des nucléotides et une polymérase capable d'amplifier ladite molécule d'acide nucléique avec un indicateur métallochromique et un ion métallique de transition ou de post-transition, ledit indicateur métallochromique et ledit ion métallique de transition ou de post-transition formant un complexe, mais ne formant pas de complexe avec le magnésium;
(b) amplifier ladite séquence d'acide nucléique dans des conditions appropriées pour obtenir un produit d'amplification de la séquence d'acide nucléique;
(c) détecter un changement dans les propriétés spectrales du complexe résultant de l'amplification de ladite séquence d'acide nucléique, la formation dudit complexe comprenant ledit indicateur métallochromique et ledit ion métallique de transition ou de post-transition entraînant un changement de couleur de la solution;
l'amplification est réalisée par la méthode définie dans l'une des revendications 1 à 7.

12. Méthode de la revendication 11,
(i) dans lequel ledit indicateur métallochrome est le 2-(5-Bromo-2-pyridylazo)-5-[N-propyl-N-(3-sulfopropyl)amino]phénol (5-Br-PAPS) ;
(ii) dans lequel ledit ion métallique est un ion Zn²⁺; et/ou
(iii) dans lequel la modification des propriétés spectrales dudit complexe se situe dans le spectre de 380 à 740 nm de longueur d'onde.

13. Kit pour la synthèse d'une séquence d'acide nucléique par amplification isotherme à médiation de boucle (LAMP) sur une séquence d'acide nucléique matrice, comprenant
(i) une première séquence nucléotidique d'amorce externe (F3) et une deuxième séquence nucléotidique d'amorce externe (B3), chacune comprenant des nucléotides d'acide nucléique verrouillé (LNA) situés dans le premier tiers de ladite séquence nucléotidique F3 ou de ladite séquence nucléotidique B3, respectivement, ledit premier tiers étant le premier tiers d'une séquence nucléotidique orientée de 5'- à 3'- direction,
(ii) une première séquence nucléotidique d'amorce interne (FIP) et une deuxième séquence nucléotidique d'amorce interne (BIP),
(iii) une ADN polymérase catalysant une réaction de type déplacement de brin pour synthétiser un brin d'acide nucléique complémentaire à partir de l'acide nucléique matrice,
(iv) des nucléotides servant de substrats à ladite ADN polymérase, et éventuellement
(v) un indicateur métallochrome, de préférence 5-Br-PAPS, PAR ou Zincon; et/ou éventuellement
(vi) des ions métalliques, de préférence des ions Zn²⁺.

14. Utilisation du kit de la revendication 13 pour la mise en oeuvre de la méthode de l'une des revendications 1 à 12.
